# EUROPÄISCHE PATENTSCHRIFT

(11) **EP 1 099 678 B1**
(45) Veröffentlichungstag und Bekanntmachung des Hinweises auf die Patenterteilung: **19.03.2003**
(21) Anmeldenummer: 00121488.1
(22) Anmeldetag: 29.09.2000
(51) Int. Cl.: C07C 45/50, B01J 31/24

(54) **Verfahren zur katalytischen Herstellung von Aldehyden aus Olefinen unter Einsatz von Ligandenmischungen**
Process for the catalytic preparation of Aldehydes from olefins using ligand mixtures
Procédé de préparation catalytique d'aldéhydes à partir d'oléfines en utilisant des mélanges des ligands

(30) Priorität: 12.11.1999 DE 19954510
(43) Veröffentlichungstag der Anmeldung: 16.05.2001
(73) Patentinhaber: Oxeno Olefinchemie GmbH, 45764 Marl (DE)
(72) Erfinder: Börner, Armin, Prof. Dr., 18059 Rostock (DE); Hess, Dieter, Dr., 45770 Marl (DE); Röttger, Dirk, Dr., 45657 Recklinghausen (DE); Selent, Detlef, Dr., 10318 Berlin (DE)

(56) Entgegenhaltungen:
- WO-A-97/20795
- WO-A-98/43935

## Beschreibung

Die Erfindung betrifft ein Verfahren zur Herstellung von Aldehyden durch Hydroformylierung von Olefinen oder Olefingemischen in Anwesenheit eines Katalysators, bestehend aus einem Metall der 8. Nebengruppe, einem Phosphitliganden und einem funktionalisierten Phosphonitliganden.

Aldehyde, insbesondere solche mit 4 bis 25 C-Atomen, können durch katalytische Hydroformylierung (oder Oxo-Reaktion) von um ein Kohlenstoffatom kürzeren Olefinen hergestellt werden. Die Hydrierung dieser Aldehyde ergibt Alkohole, die zum Beispiel zur Herstellung von Weichmachern oder als Detergentien genutzt werden. Die Oxidation der Aldehyde liefert Carbonsäuren, die beispielsweise zur Herstellung von Trocknungsbeschleunigern für Lacke oder als Stabilisatoren für PVC verwendet werden können.

Die Art des Katalysatorsystems und die optimalen Reaktionsbedingungen für die Hydroformylierung sind von der Reaktivität des eingesetzten Olefins abhängig. Einen kompakten Überblick über die Hydroformylierung, Beispiele für Katalysatoren und ihre Einsatzgebiete, gängige großtechnische Prozesse usw. finden sich in B. Cornils, W. A. Herrmann (Ed.), "Applied Homogeneous Catalysis with Organometallic Compounds", VCH, Weinheim, New-York, Basel, Cambridge, Tokyo, **1996**, Vol.1, S. 29-104. Die Abhängigkeit der Reaktivität der Olefine von ihrer Struktur ist unter anderem von J. Falbe, "New Syntheses with Carbon Monoxide", Springer-Verlag, Berlin, Heidelberg, New York, **1980,** S. 95 ff., beschrieben. Die unterschiedliche Reaktivität von isomeren Octenen ist ebenfalls bekannt (B. L. Haymore, A. van Hasselt, R. Beck, Annals ofthe New York Acad. Sci., 415 (1983), S. 159-175).

Über die unterschiedlichen Prozesse und Katalysatoren ist eine Vielzahl von Olefinen für die Oxierung zugänglich. Ein mengenmäßig bedeutender Rohstoff ist Propen, aus dem n- und i-Butyraldehyd erhalten werden.

Technische Olefingemische, die als Edukte für die Oxo-Synthese verwendet werden, enthalten oftmals Olefine der verschiedensten Strukturen mit unterschiedlichen Verzweigungsgraden, unterschiedlicher Lage der Doppelbindung im Molekül und gegebenenfalls auch unterschiedlichen C-Zahlen. Ein typisches Beispiel ist Raffinat I als Mischung der C₄-Olefine 1-Buten, 2-Buten und Isobuten. Besonders gilt dies für Olefingemische, die durch Di-, Tri- oder weitergehende Oligomerisierung von C₂-C₅-Olefinen oder anderen leicht zugänglichen höheren Olefinen bzw. durch Cooligomerisierung von Olefinen entstanden sind. Als Beispiele für technische Olefingemische, die durch Hydroformylierung zu den entsprechenden Aldehydgemischen umgesetzt werden können, seien Tri- und Tetrapropene sowie Di-, Tri- und Tetrabutene genannt.

Die Produkte der Hydroformylierung sind durch die Struktur der Einsatzolefine, das Katalysatorsystem und die Reaktionsbedingungen bestimmt. Unter Bedingungen, bei denen es zu keiner Verschiebung der Doppelbindung im Olefin kommt, im Folgenden als nicht-isomerisierende Bedingungen bezeichnet, wird die Formylgruppe an der Stelle im Molekül eingeführt, an der sich die Doppelbindung befand, wobei wiederum zwei unterschiedliche Produkte entstehen können. So kann beispielsweise bei der Hydroformylierung von 1-Penten Hexanal und 2-Methylpentanal gebildet werden. Bei der Hydroformylierung von 1- Penten unter isomerisierenden Bedingungen, bei der neben der eigentlichen Oxierung auch eine Verschiebung der Doppelbindung im Olefin stattfindet, würde zusätzlich 2-Ethylbutanal als Produkt zu erwarten sein.

Sind als Folgeprodukt der Oxoaldehyde Alkohole für die Herstellung von Detergentien und Weichmacher angestrebt, so sollen durch die Oxoreaktion möglichst linerare Aldehyde hergestellt werden. Die hieraus synthetisierten Produkte weisen besonders vorteilhafte Eigenschaften auf, z. B. niedrige Viskositäten der daraus hergestellten Weichmacher.

Die oben genannten technischen Olefingemische enthalten oftmals nur geringe Anteile an Olefinen mit endständiger Doppelbindung. Um aus ihnen Produkte herzustellen, in denen mehr terminal oxiertes Olefin vorliegt als im ursprünglichen Olefingemisch, muß unter isomerisierenden Bedingungen hydroformyliert werden. Geeignete Verfahren dafür sind beispielsweise Hochdruck-Hydroformylierungen mit Kobaltkatalysatoren. Diese Verfahren haben jedoch den Nachteil, daß relativ viel Nebenprodukte, zum Beispiel Alkane, Acetale oder Ether gebildet werden.

Bei der Verwendung von Rhodiumkomplexen als Katalysator ist der Ligand für die Produktzusammensetzung der Aldehyde mitbestimmend. Nichtmodifizierte Rhodiumcarbonylkomplexe katalysieren die Hydroformylierung von Olefinen mit endständigen und innenständigen Doppelbindungen, wobei die Olefine auch verzweigt sein können, zu Aldehyden mit einem hohen Verzweigungsgrad. Der Anteil an terminal oxiertem Olefin ist, im Vergleich zum Kobalt-oxierten Produkt, deutlich geringer.

Mit einem ligandmodifizierten Katalysatorsystem, bestehend aus Rhodium und Triorganophosphin, z. B. Triphenylphosphin werden α-Olefine mit hoher Selektivität terminal hydroformyliert. Eine Isomerisierung der Doppelbindungen und/oder die Hydroformylierung der innenständigen Doppelbindungen tritt kaum auf.

Die Oxierung von Olefinen mit innenständigen Doppelbindungen an Katalysatorsystemen, die sterisch anspruchsvolle Phosphitliganden enthalten, erfolgt nicht zufriedenstellend bei hohen Umsätzen mit gleichzeitig hoher n/iso-Selektivität.

Eine Übersicht über den Einfluß von Liganden auf die Aktivität und Selektivität bei der Hydroformylierung findet sich im oben zitierten Buch von B. Cornils und W. A. Herrmann.
Verglichen mit Phosphin- oder Phosphitliganden finden sich in der Fachliteratur nur wenige Publikationen zum Einsatz von Phosphonigsäurediestern (im Folgenden Phosphonit genannt) als Liganden in Oxierungsreaktionen. In JP-OS Hei 9-268152, WO 98/43935 und in JP-AS Hei 9-255610 werden Katalysatorsysteme, bestehend aus Rhodium, einem Triorganophosphonit-Liganden oder einem zweizähnigen Phosphonitliganden für die Hydroformylierung von acyclischen oder cyclischen Olefinen bzw. Olefingemischen beschrieben. Die Hydroformylierung von Olefinen mit innenständigen Doppelbindungen ist jedoch nicht offenbart. Weiterhin finden sich keine Angaben über die Struktur der Produkte, insbesondere über das Verhältnis von innenständiger zu terminaler Oxierung.

WO 97/20795 beschreibt ein Hydroformylierungsverfahren, bei dem Metallorganophosphite und sterisch gehinderte Organophosphorliganden eingesetzt werden. Diese Ligandenkombination soll durch die unterschiedliche katalytische Aktivität der einzelnen Liganden als Indikator für die Aktivität des Gesamtsystems dienen. Phosphonitliganden sind in WO 97/20795 nicht beschrieben.

Es ist weiterhin z. B. durch EP 0 214 622 bekannt, mehrzähnige Polyphosphitliganden als Bestandteil von Hydroformylierungskatalysatoren einzusetzen. Hier werden auch verschiedene Liganden gleichzeitig verwendet. Die Auswirkungen von Ligandenmischungen auf die Linearität des Produkts sind nicht beschrieben; insbesondere ist eine erwünschte Steuerung der Reaktion zu linearen Aldehyden nicht offenbart.

Aufgabe der vorliegenden Erfindung war es daher, ein Verfahren zur Hydroformylierung von Olefinen bereitzustellen, mit dem verzweigte, unverzweigte, end- oder innenständige Olefine mit hohen Ausbeuten und Selektivitäten terminal oxiert werden können, d.h. möglichst lineare Aldehyde hergestellt werden können. Es wurde überraschend gefunden, daß die Hydroformylierung von Olefinen unter Katalyse von Metallen der 8. Nebengruppe mit Phosponiten, - arsenoniten und -stibenoniten in Gegenwart von Organophosphiten mit hohen Ausbeuten und Selektivitäten zu linearen, endständig oxierten Produkten führt.

Gegenstand der vorliegenden Erfindung ist daher ein Verfahren zur Herstellung von Aldehyden mit 4 bis 25 Kohlenstoffatomen durch katalytische Hydroformylierung der entsprechenden Olefine, wobei als Katalysator ein Metall der 8. Nebengruppe des Periodensystems in Gegenwart eines Liganden A der Formel I 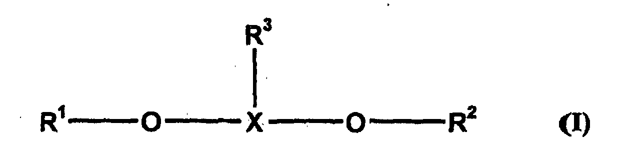 mit X = As, Sb oder P und
- R¹, R², R³:: substituierter oder unsubstituierter aliphatischer, cycloaliphatischer oder aromatischer Kohlenwasserstoffrest mit 1 bis 50 Kohlenstoffatomen,
wobei zwei der Reste R¹, R², R³ eine kovalente Verknüpfung aufweisen können, mit der Maßgabe, daß wenigstens einer der Kohlenwasserstoffreste R¹, R², R³ ein Heteroatom aus der Gruppe O, S, N, F, Cl, Br, I, Se und Te enthält, und eines Liganden B der Formel II 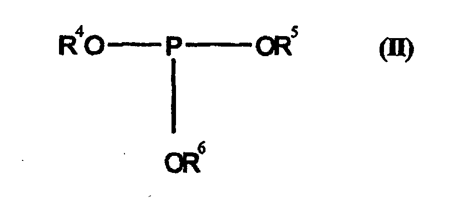
- mit R⁴, R⁵, R⁶:: substituierter oder unsubstituierter aliphatischer oder aromatischer Kohlenwasserstoff mit 1 bis 50 Kohlenstoffatomen,
wobei je zwei der Reste R⁴, R⁵ und R⁶ eine kovalente Verknüpfung aufweisen können, verwendet wird.

Das erfindungsgemäße Verfahren ermöglicht die Herstellung von weitgehend endständigen Aldehyden, d. h. Produkte mit einem hohen n : iso-Verhältnis bei gleichzeitig guten Gesamtumsätzen. Dies bedeutet eine deutliche Verbesserung der bekannten Verfahren, die entweder gute Ausbeuten mit mäßigen n : iso-Selektivitäten oder mäßige Ausbeuten mit guten n : iso-Selektivitäten zeigen.

Das einem Aldehyd entsprechende Olefin wird durch das erfindungsgemäße Verfahren um ein Kohlenstoffatom verlängert; bzw. das entsprechende Olefin weist ein Kohlenstoffatom weniger als der Aldehyd auf.

Die im erfindungsgemäßen Verfahren eingesetzten Liganden A werden im Folgenden jeweils als heterofunktionalisierte Phosphonite, Arsenonite oder Stibenonite bezeichnet. Unter diesen heterofunktionalisierten Phosphoniten, Arsenoniten oder Stibenoniten werden Verbindungen mit einem Atom der Hauptgruppe des Periodensystems (P, As, Sb) verstanden, das ein freies Elektronenpaar und zwei Einfachbindungen zu jeweils einem Sauerstoffatom und eine Einfachbindung zu einem Kohlenstoffatom besitzt. Die allgemeinen Formeln I, III, IV, V, VI und VII sowie die Beispiele der Tabelle 1 zeigen mögliche Liganden A für das erfindungsgemäße Verfahren.

Die Liganden A enthalten neben dem Atom der 5. Hauptgruppe mindestens ein weiteres Heteroatom mit mindestens einem freien Elektronenpaar. Das Atom der fünften Hauptgruppe und das weitere Heteroatom sind im Ligand A derart angeordnet, daß ein Metallatom intramolekular an diesen beiden Atomen gleichzeitig koordiniert sein kann. Die trifft zum Beispiel zu, wenn ein Phosphoratom, ein Heteroatom und die dazwischenliegenden Atome mit dem koordinierten Metallatom einen 4 bis 15-gliedrigen Ring, vorzugsweise einen 4-9 gliedrigen Ring bilden können.
Diese Heteroatome können Sauerstoff, Schwefel, Stickstoff, Fluor, Chlor, Brom, Jod, Selen oder Tellur sein. Diese Heteroatome können in funktionellen Gruppen wie z.B. Ethern, Thioethern und tertiären Aminen enthalten sein und/oder Teil einer Kohlenstoffkette oder eines Ringes sein. Es ist auch möglich, daß die Liganden A mehr als ein Heteroatom enthalten, das diesen Forderungen entspricht. Die erfindungsgemäß eingesetzten Liganden A sollten eine koordinative Bindung zwischen Heteroatom und Metall ausbilden können, die eine geringere Stärke besitzt, als die zwischen dem Atom der fünften Hauptgruppe, d.h. P, As, Sb und dem Metall.

In der Fachliteratur werden Liganden, die neben einer starken Wechselwirkung zu einem Metall eine zweite, aber deutlich schwächere (labile) Wechselwirkung aufweisen, oft als hemilabile Liganden bezeichnet (Übersichtsartikel: A. Bader, E. Linder, Coord. Chem. Rev. **1991**, 108, 27-110; C. S. Slone, D. A. Weinberger, C. A. Mirkin, Prog. Inorg. Chem. **1999**, 48, 233). Für einige Literaturbeispiele konnte anhand von Röntgenstrukturen die zweite, schwächere Wechselwirkung des Liganden, d. h. des Heteroatoms mit dem Metall nachgewiesen werden. Für die vorliegenden heterofunktionalisierten Liganden A ist das Koordinationsverhalten nicht bekannt, es kann aber aus sterischen Überlegungen geschlossen werden, daß jeweils eine Koordination des Metallatoms über z. B. ein Phosphoratom und ein Heteroatom besteht.

Als katalytisch aktives Metall kommen die Metalle der 8. Nebengruppe des Periodensystems der Elemente in Frage, wie Rhodium, Kobalt, Platin oder Ruthenium.

Der aktive Katalysatorkomplex für die Hydroformylierung wird im erfindungsgemäßen Verfahren aus einem Salz oder einer Verbindung des Metalls der 8. Nebengruppe des Periodensystems (Katalysatorvorläufer), den Liganden A und B, Kohlenmonoxid und H₂ gebildet, zweckmäßig geschieht dies in situ während der Hydroformylierung. Diese Komponenten des Katalysators können in den Prozeß gleichzeitig oder nebeneinander eingeschleust werden; der Katalysatorkomplex bildet sich dann mit dem Synthesegas. Übliche Katalysatorvorläufer sind beispielsweise Octanoate, Nonanoate oder Acetylacetonate. Das molare Verhältnis zwischen Metall und den Liganden A und B liegt jeweils bei 1 : 1 bis 1 : 100, bevorzugt zwischen 1 : 1 und 1 : 50. In der Praxis haben sich Metall/Ligand-Verhältnisse von 1 : 5, 1 : 10 oder 1 : 20 bewährt. Die Konzentration des Metalls im Reaktionsgemisch liegt im Bereich von 1 ppm bis 1000 ppm, vorzugsweise im Bereich 5 ppm bis 300 ppm. Die Reaktionstemperaturen des erfindungsgemäßen Verfahrens liegen zwischen 60 °C und 180 °C, vorzugsweise zwischen 90 °C und 150 °C, die Drücke betragen 1-300 bar, vorzugsweise 10-60 bar.

Die im erfindungsgemäßen Verfahren eingesetzten Liganden A können Strukturen gemäß den Formel I, III, IV, V, VI oder VII aufweisen.

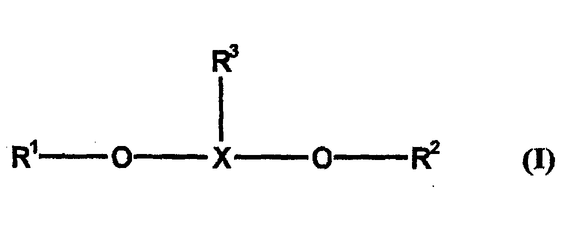

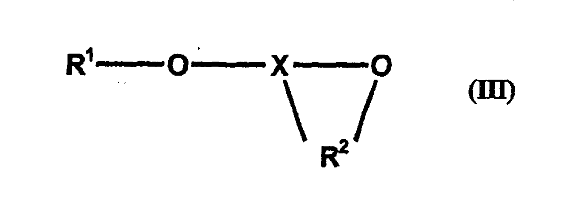

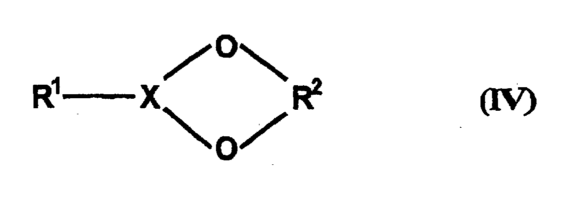

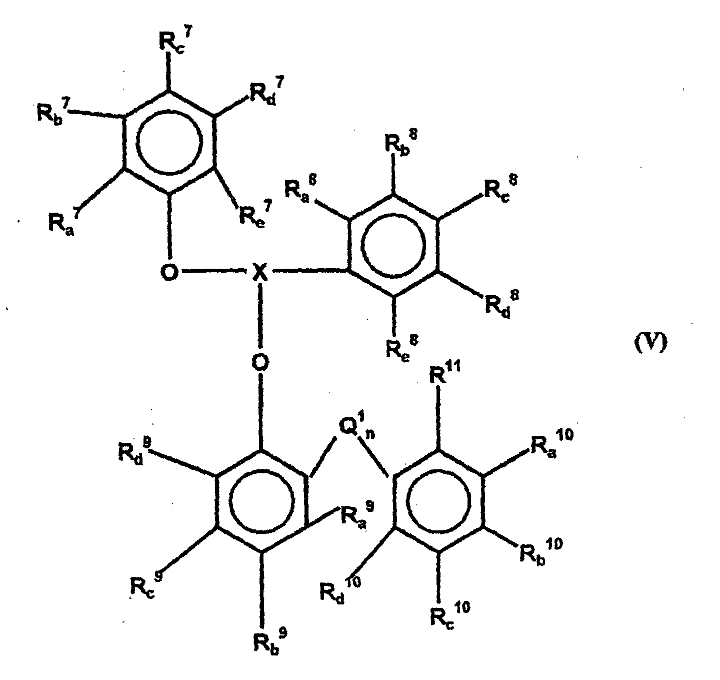

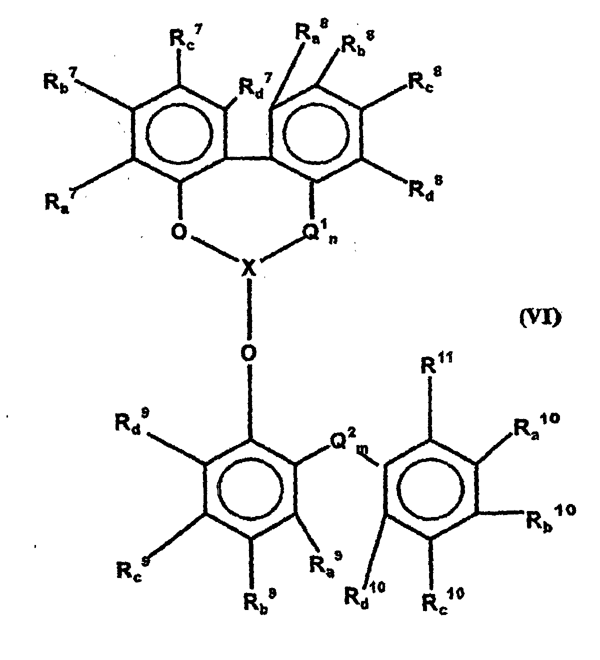

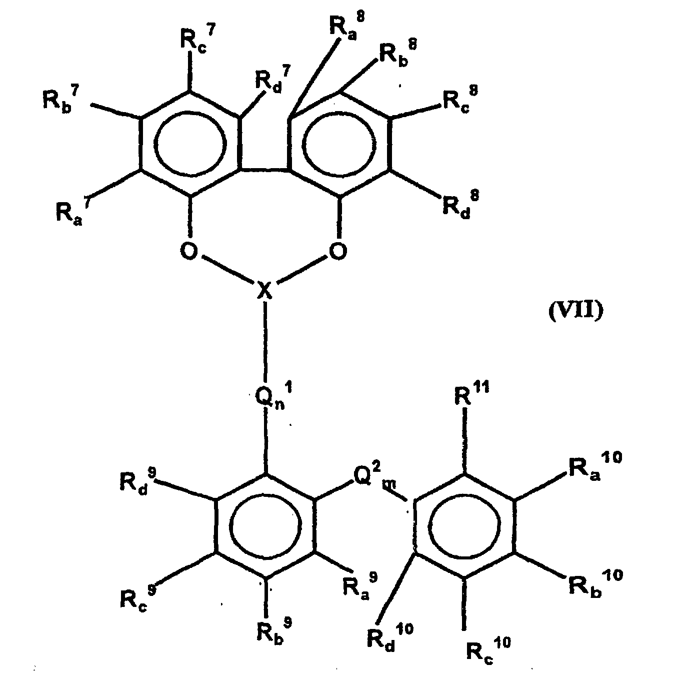

Die Reste R¹, R² und R³ stehen in diesen Formeln jeweils für einen substituierten oder unsubstituierten, aliphatischen, cycloaliphatischen oder aromatischen Kohlenwasserstoffrest mit jeweils 1 bis 50 Kohlenstoffatomen, mit der Maßgabe, daß wenigstens einer der Kohlenwasserstoffreste R¹, R² oder R³ ein Heteroatom aus der Gruppe O, S, N, F, Cl, Br, I, Se und Te enthält. Jeweils zwei der Reste R¹, R² und R³ können eine kovalente Verknüpfung zueinander aufweisen. Der Rest R² bezeichnet in den Formeln m und IV selbstverständlich zweiwertige Kohlenwasserstoffreste.

Die Reste R⁷ₐ₋ₑ, R⁸ₐ₋ₑ, R⁹_{a-d} und R¹⁰_{a-d} stehen für H, einen aliphatischen oder aromatischen Kohlenwasserstoffrest oder für eine aliphatische oder aromatische Alkoxygruppe, jeweils mit 1 bis 25 Kohlenstoffatomen, wobei die Substituenten mit den Indices a-e jeweils für gleiche oder verschiedene Substituenten stehen können.

Beispiele für Liganden A mit einsetzbaren Substituentenmustern sind in Tabelle 1 aufgeführt.

R¹¹ steht für -O-R¹², -CH₂-O-R¹², -COOR¹², -COOM, -SR¹², -NR¹²R¹³, -CH₂NR¹²R¹³, -CH₂CO₂M, -N=CR¹²R¹³, wobei R¹² und R¹³ gleich oder verschieden sein können und die gleiche Bedeutung wie R⁷ₐ besitzen und M = H, Li, Na, K, NH₄ bedeuten.

Beispiele für die Reste R⁷ ₐ₋ₑ, R⁸ ₐ₋ₑ, R⁹ _{a-d} und R¹⁰ _{a-d} sind : H, t.-Butylgruppe, Methoxygruppe, Ethoxygruppe, t-Butylethergruppe, iso-Propylgruppe und t-Amylgruppe.

X bezeichnet ein Phosphor-, Arsen- oder Antimonatom.

Q¹ und Q² stehen jeweils für einen Methylenrest oder eine Gruppe der Formel CR¹⁴R¹⁵, wobei R¹⁴ und R¹⁵ gleich oder verschieden sind und die gleiche Bedeutung wie R⁷ₐ besitzen. Die Indices n und m stehen jeweils für 0 oder 1.

Liganden des Typs A, die im erfindungsgemäßen Verfahren eingesetzt werden können, sind beispielsweise:

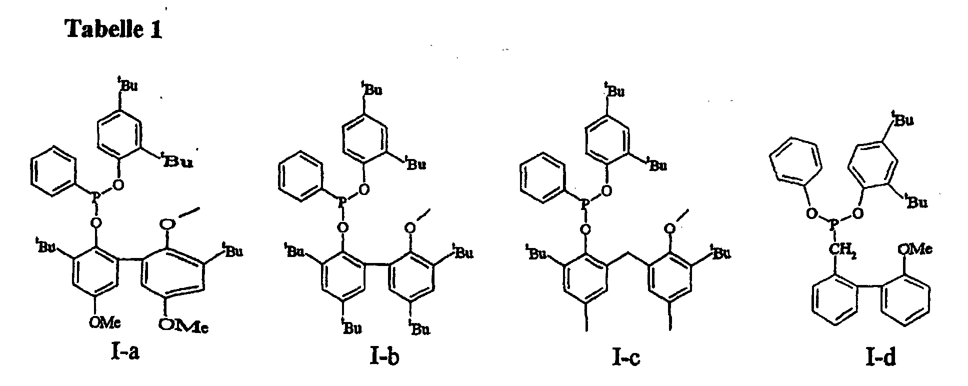

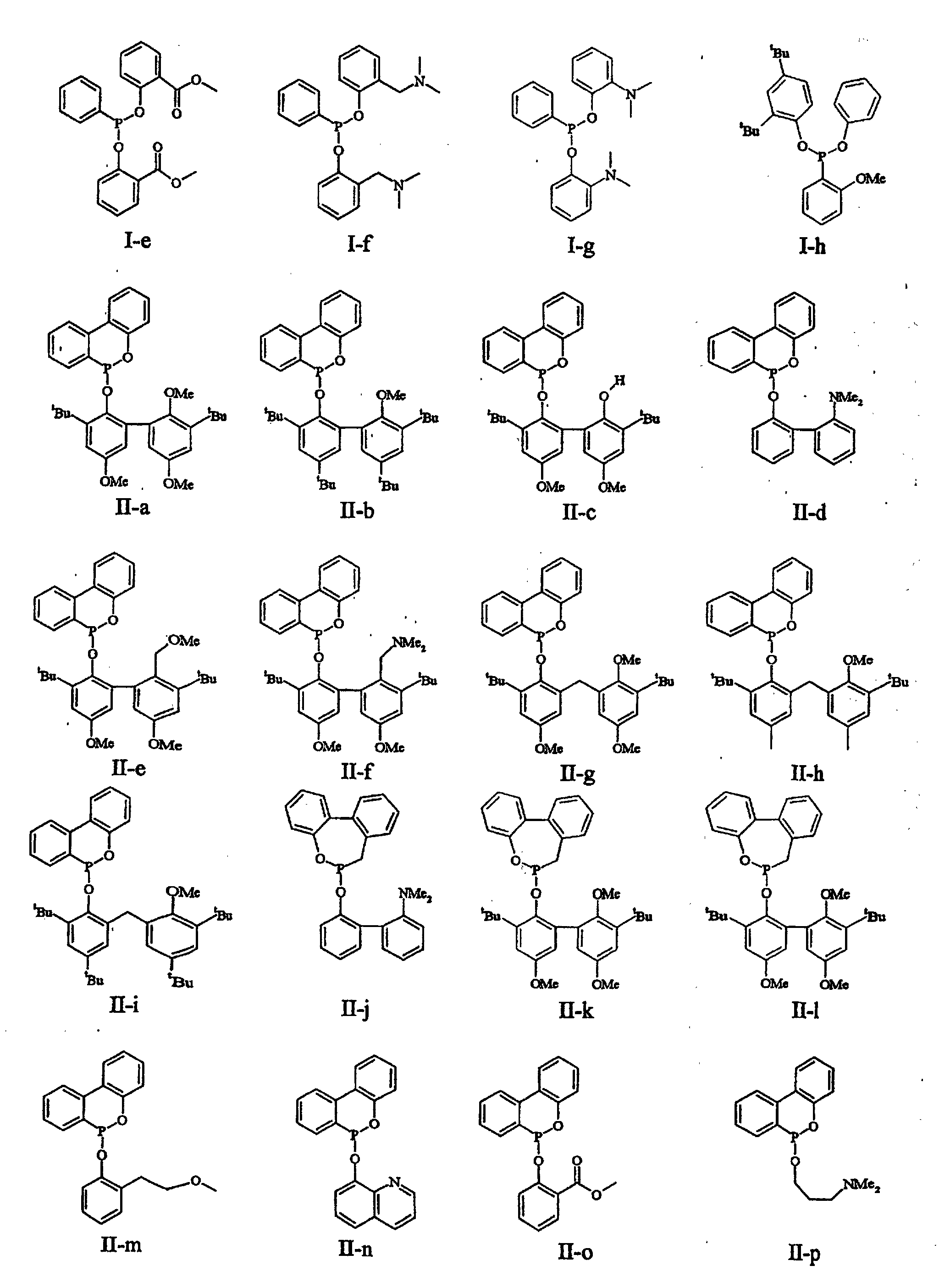

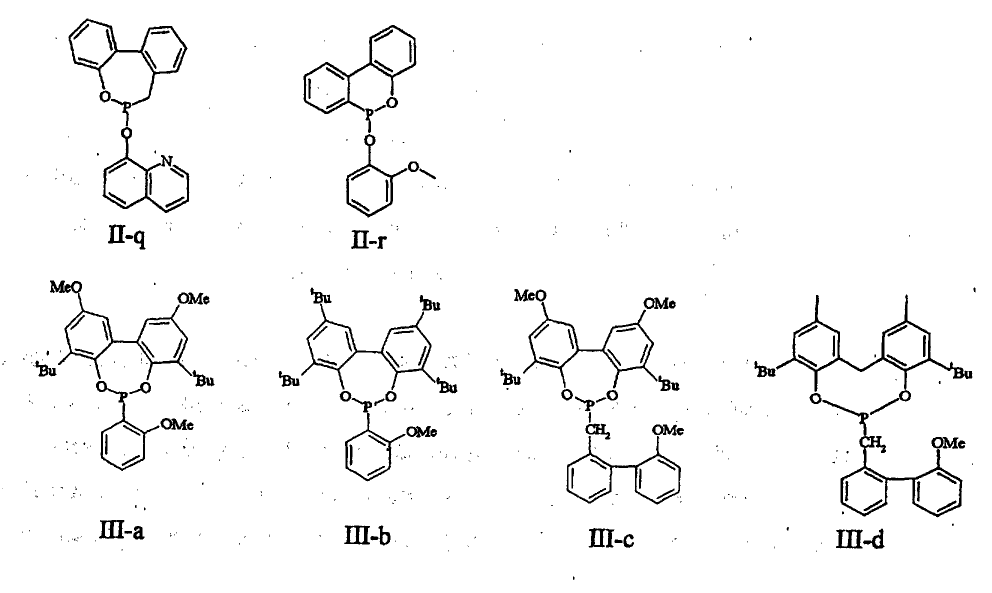

Im erfindungsgemäßen Verfahren können als Ligand B eingesetzt werden:

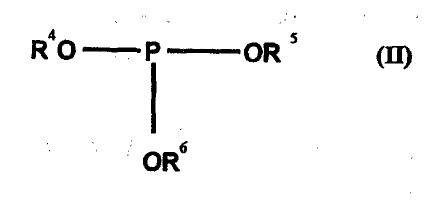

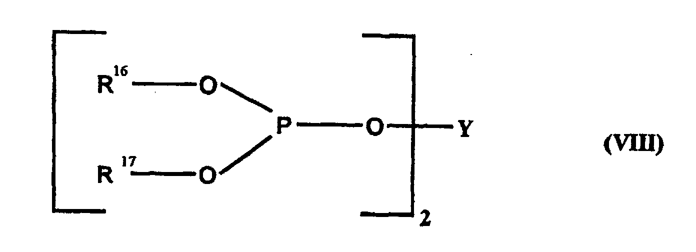

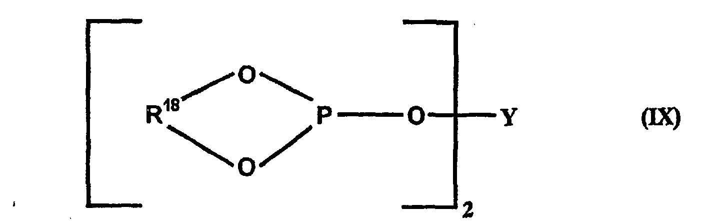

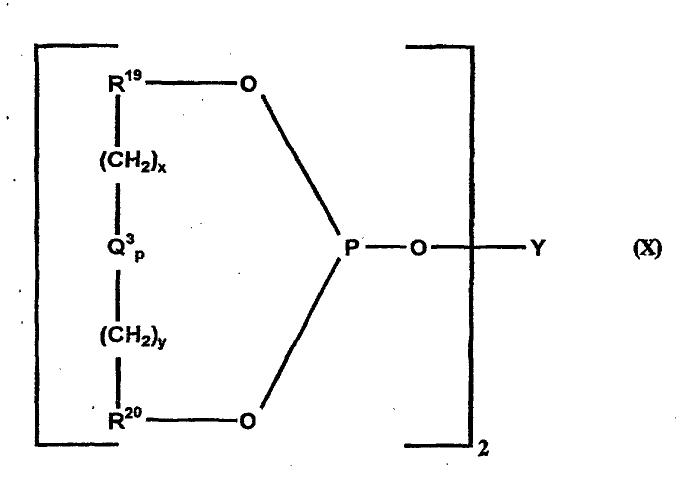

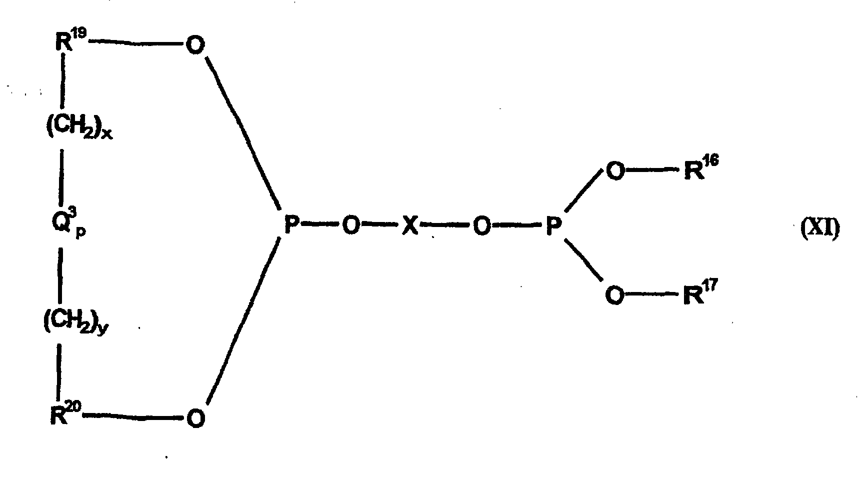

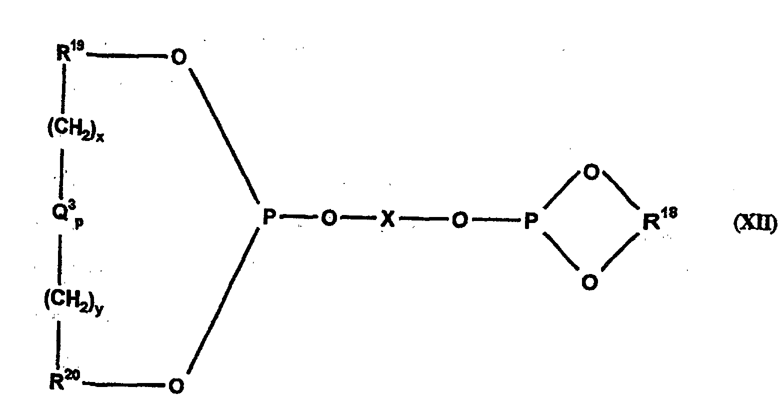

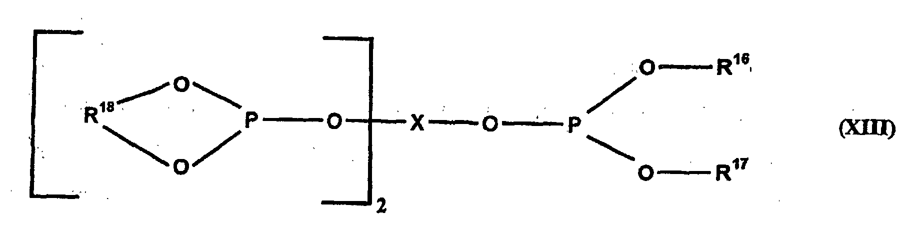

Die Reste bzw. Substituenten stehen für
- R¹⁶, R¹⁷:: aliphatischer oder aromatischer, substituierter oder unsubstituierter Kohlenwasserstoffrest mit 1 bis 50 Kohlenstoffatomen, wobei R¹⁶ und R¹⁷ gleich oder verschieden sein können und
- R¹⁸:: aliphatischer oder aromatischer, substituierter oder unsubstituierter Kohlenwasserstoffrest mit 1 bis 50 Kohlenstoffatomen und
- R¹⁹, R²⁰:: xaliphatischer oder aromatischer, substituierter oder unsubstituierter Kohlenwasserstoffrest mit 1 bis 50 Kohlenstoffatomen, wobei R¹⁹ und R²⁰ gleich oder verschieden sein können,
- Q³:: C(R²¹)₂, O, S, NR²¹, Si(R²¹)₂, CO, wobei R²¹ = H, substituierter oder unsubstituierter aliphatischer oder aromatischer Kohlenwasserstoffrest mit 1 bis 50 Kohlenwasserstoffatomen,
- x, y, p:: jeweils ganze Zahlen 0 bis 5
- Y:: aliphatischer oder aromatischer, substituierter oder unsubstituierter Kohlenwasserstoffrest mit 1 bi 50 Kohlenstoffatomen.

Beispiele für die Reste Y, R¹⁶, R¹⁷, R¹⁸, R¹⁹, R²⁰ sind z. B. Methyl-, Ethyl-, Propyl-, Butyl-, Aryl, Phenyl-, oder Naphthylreste. Diese Reste können jeweils unsubstituiert oder z. B. mit Nitro-, Carboxylat-, Carbonyl-, Cyano-, Amino-, Hydroxyl-, Sulfonyl-, Silyl-, Acyl-, Alkyl- oder Arylgruppen sowie Halogenen substituiert sein.
R¹⁶ und R¹⁷ stehen für einwertige, Y, R¹⁸, R¹⁹, R²⁰ und Q³ für zweiwertige Reste der o. g. Gruppen.

Liganden dieses Typs sind bekannt; ihre Herstellung und Verwendung kann z. B. in den US-Patenten 4,668,651; 4,748,261; 4,769,498; 4,774,361; 4,885,401; 5,113,022, 5,179,055; 5,202,297; 5,235,113; 5,254,741; 5,264,616; 5,312,996; 5,364,950 und 5,391,801 nachgelesen werden.

Als bevorzugte Liganden des Typs B können die folgenden Verbindungen eingesetzt werden:

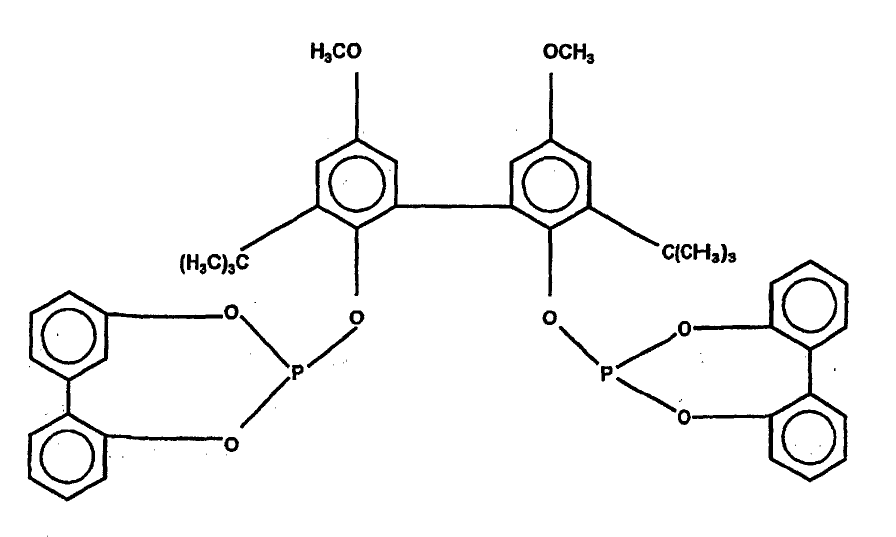

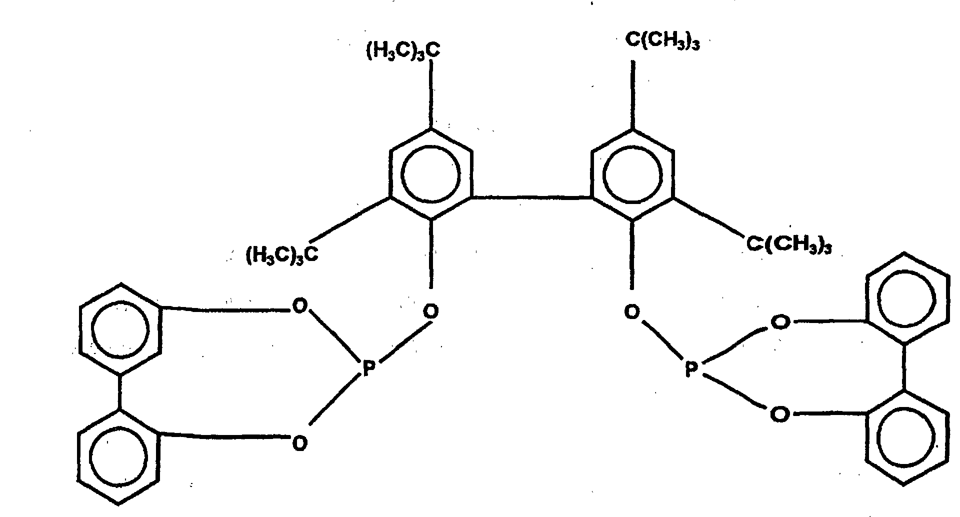

Der Katalysator, d. h. Metall der 8. Nebengruppe und Ligand A und B können homogen im Hydroformylierungsgemisch, bestehend aus Edukt (Olefin) und Produkt (Aldehyde, Alkohole, Hochsieder), gelöst werden. Optional kann zusätzlich ein Lösungsmittel wie z. B. Alkohole oder Aldehyde, hier wieder bevorzugt die Produkte der Hydroformylierung, zugesetzt werden.

Die Edukte für eine Hydroformylierung gemäß dem Verfahren der Erfindung sind Olefine oder Gemische von Olefinen, insbesondere Monoolefine mit 4 bis 25, bevorzugt 4 bis 16, besonders bevorzugt 4 bis 8 Kohlenstoffatomen mit end- oder innenständigen C-C-Doppelbindungen, wie z. B. 1- oder 2-Penten, 2-Methylbuten-1, 2-Methylbuten-2, 3-Methylbuten-1, 1-,2- oder 3-Hexen, das bei der Dimerisierung von Propen anfallende C₆-Olefingemisch (Dipropen), Heptene, 2- oder 3-Methyl-1-hexen, Octene, 2-Methylheptene, 3-Methylheptene, 5-Methylhepten-2, 6-Methylhepten-2, 2-Ethylhexen-1, das bei der Dimerisierung von Butenen anfallende isomere C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge. Ebenfalls können Olefine oder Olefingemische, die durch Fischer-Tropsch-Synthese erzeugt worden sind, sowie Olefine, die durch Oligomerisierung von Ethen erhalten wurden oder Olefine, die über Methathesereaktionen zugänglich sind, eingesetzt werden. Bevorzugte Edukte sind C₄-, C₈-, C₉-, C₁₂- oder C₁₆-Olefingemische.

Mit dem erfindungsgemäßen Verfahren können unter Verwendung der heterofunktionalisierten Liganden α-Olefine, verzweigte, innenständige und innenständig verzweigte Olefine mit hohen Raum-Zeit-Ausbeuten hydroformyliert werden. Bemerkenswert ist dabei die hohe Ausbeute an endständig hydroformyliertem Olefin, selbst wenn im Edukt nur ein geringer Anteil an Olefinen mit endständiger Doppelbindung vorhanden war.

Die im erfindungsgemäßen Verfahren eingesetzten Ligandenmischungen aus den Liganden A und B zeigen einen deutlichen synergistischen Effekt und liefern auch bei verzweigten Olefinen als Edukt bei guten Ausbeuten Produkte mit hoher Linearität, d. h. ein gutes n : i-Verhältnis der Produkte.

Die folgenden Beispiele sollen die Erfindung weiter erläutern, nicht aber ihren Anwehdungsbereich beschränken, der sich aus den Patentansprüchen ergibt.

Exemplarisch wurden Hydroformylierungen von verschiedenen Olefinen mit den folgenden Liganden durchgeführt:

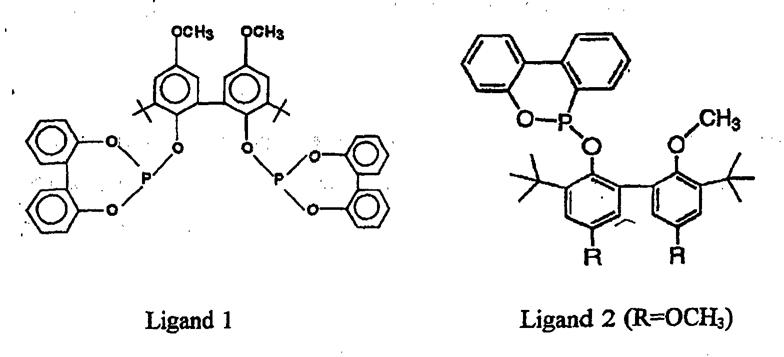

Zum Vergleich mit dem erfindungsgemäßen Verfahren wurde eine Mischung des Liganden 1 mit Tris-(2,4-di-tert.-butylphenyl)-phosphit (TDTBPP, Ligand 3) untersucht.

Ligand 1 entspricht dem Ligandentyp B, Ligand 2 dem Ligandentyp A. Alle %-Angaben sind Gew.-%.
Die Versuche wurden in 300 ml-Laborautoklaven der Fa. Berghof durchgeführt. Hierzu wurden 60 g Olefin + 40 g Texanol im Autoklaven vorgelegt. Der / die Ligand(en) und der Rh-Precursor wurden in 60 g Texanol gelöst und beim Versuchsstart über eine Druckpipette zudosiert. Als Katalysatorprecursor wurde 0,135 g Rhodiumnonanoat eingesetzt (entspricht 200 mg/kg Rh). Synthesegas (50 Vol.-% H_{2,} 50 Vol.-% CO) wurde über einen Begasungsrührer so eindosiert, daß der Druck konstant gehalten wurde. Die Reaktionsdauer betrug 8 h, die Rührerdrehzahl 1000 Umdrehungen/min. Die Versuche wurden bei 140 °C und 20 bar durchgeführt.

### Versuch 1

In einem Versuch mit 200 ppm Rh, Ligand 1 (P:Rh=10:1) und 60 g 2,4,4-Trimethylpenten-2, einem verzweigten Olefin mit innenständiger Doppelbindung wurde bei 140 °C und 20 bar nach 8 Stunden nur 1,6 % 3,5,5-Trimethylhexanal gebildet.

### Versuch 2

In einem Versuch mit 200 ppm Rh, Ligand 1 (P:Rh=10:1) und 60 g 2,4,4-Trimethylpenten-1 wurde bei 140 °C und 20 bar nach 8 Stunden ein Umsatz zu 3,5,5-Trimethylhexanal von 76,5 % erzielt.

### Versuch 3

In einem Versuch mit 200 ppm Rh, Ligand 1 (P:Rh=10:1) und 60 g eines Gemisches unverzweigter Octene mit ca. 3,3 % Gehalt an 1-Octen wird bei 140 °C und 20 bar nach 8 Stunden ein Umsatz zu n-Nonanal von 91,9 % erzielt. Mit dem Liganden erhält man somit eine ausgezeichnete Selektivität zu 1-Nonanal.

### Versuch 4

In einem Versuch mit 200 ppm Rh, Ligand 2 (P:Rh=10:1) und 60 g 2,4,4-Trimethylpenten-2 wird nur ein Umsatz zu 3,5,5-Trimethyl-hexanal von 11,3 % erzielt, aber das Produktgemisch enthält 63,8 % 2,4,4-Trimethylpenten-1.

### Versuch 5

In einem Versuch mit 200 ppm Rh, Ligand 2 (P:Rh=10:1) und 60 g 2,4,4-Trimethylpenten-1 wird ein Umsatz zu 3,5,5-Trimethylhexanal von 46,4 % erzielt.

### Versuch 6

In einem Versuch mit 200 ppm Rh, Ligand 2 (P:Rh=10:1) und 60 g eines Gemisches unverzweigter Octene mit ca. 3,3 % Gehalt an 1-Octen wird bei 140 °C und 20 bar ein Umsatz zu n-Nonanal von 27,7 % bei einem Gesamtumsatz von ca. 70 % erzielt.

### Versuch 7

Mit 200 ppm Rh, dem Liganden 1 (P:Rh-Verhältnis 10:1) und 60 g Di-n-Buten als Olefin erhält man im Versuchsprodukt nach 8 Stunden Reaktionszeit bei einem Umsatz von 40 % ein n/iso-Verhältnis von 3,53.

### Versuch 8

Mit 200 ppm Rh, dem Liganden 2 (P:Rh-Verhältnis 10:1) und 60 g Di-n-Buten als Olefin erhält man bei einem im Versuchsprodukt nach 8 Stunden Reaktionszeit bei einem Umsatz von 30 % ein n/iso-Verhältnis von 0,7.

### Versuch 9

Mit 200 ppm Rh, dem Liganden 1 (P:Rh-Verhältnis 2:1) und 60 g Di-n-Buten als Olefin erhält man im Versuchsprodukt nach 8 Stunden Reaktionszeit bei einem Umsatz von 70 % ein n/iso-Verhältnis von 0,96.

### Versuch 10

Mit 200 ppm Rh, einer Mischung aus Ligand 1 und Ligand 2 (jeweils P:Rh=5:1) und 60 g Di-n-Buten als Olefin erhält man im Versuchsprodukt nach 8 Stunden Reaktionszeit ein n/iso-Verhältnis von 1,83 bei einem Umsatz von 73 %. Mit der Mischung wird überraschenderweise ein Umsatz erzielt, der wesentlich höher ist als der mit den einzelnen Liganden erzielbare Umsatz bei gleichzeitig noch hohem n/i-Verhältnis.

### Versuch 11

Mit 200 ppm Rh, einer Mischung aus Ligand 1 (P:Rh=5:1) und Ligand 2 (P:Rh=10:1) und 60 g Di-n-Buten als Olefin erhält man im Versuchsprodukt nach 8 Stunden Reaktionszeit ein n/iso-Verhältnis von 1,29 bei einem Umsatz von 83 %. Mit der Mischung wird somit bei gutem n/i-Verhältnis ein noch höherer Umsatz als in Versuch 10 erzielt.

Die Mischungen der Liganden 1 und 2 zeigen somit einen deutlichen synergistischen Effekt.

### Versuche 12 bis 14: Vergleichsbeispiele

### Versuch 12

Mit 200 ppm Rh, einer Mischung aus Ligand 1 und Ligand 3 (jeweils P:Rh=10:1) und 60 g Di-n-Buten als Olefin erhält man im Versuchsprodukt nach 8 Stunden Reaktionszeit bei einem Umsatz von 37 % ein n/iso-Verhältnis von 3,41.

### Versuch 13

Mit 200 ppm Rh, einer Mischung aus Ligand 1 (P:Rh=5:1) und Ligand 3 (P:Rh=10:1) und 60 g Di-n-Buten als Olefin erhält man im Versuchsprodukt nach 8 Stunden Reaktionszeit ein n/iso-Verhältnis von 3,61 bei einem Umsatz von nur 40%.

### Versuch 14

Mit 200 ppm Rh, einer Mischung aus Ligand 1 (P:Rh=2:1) und Ligand 3 (P:Rh=10:1) und 60 g Di-n-Buten als Olefin erhält man im Versuchsprodukt nach 8 Stunden Reaktionszeit ein n/iso-Verhältnis von 0,71 bei einem Umsatz von 93 %. Mischungen zwischen Ligand 1 und Ligand 3 verhalten sich additiv. Ein synergistischer Effekt auf den Umsatz wie bei der erfindungsgemäß eingesetzten Mischung aus Ligand 1 und Ligand 2 ist nicht zu beobachten.

Tabelle 3 zeigt eine Zusammenstellung der Versuchsergebnisse.

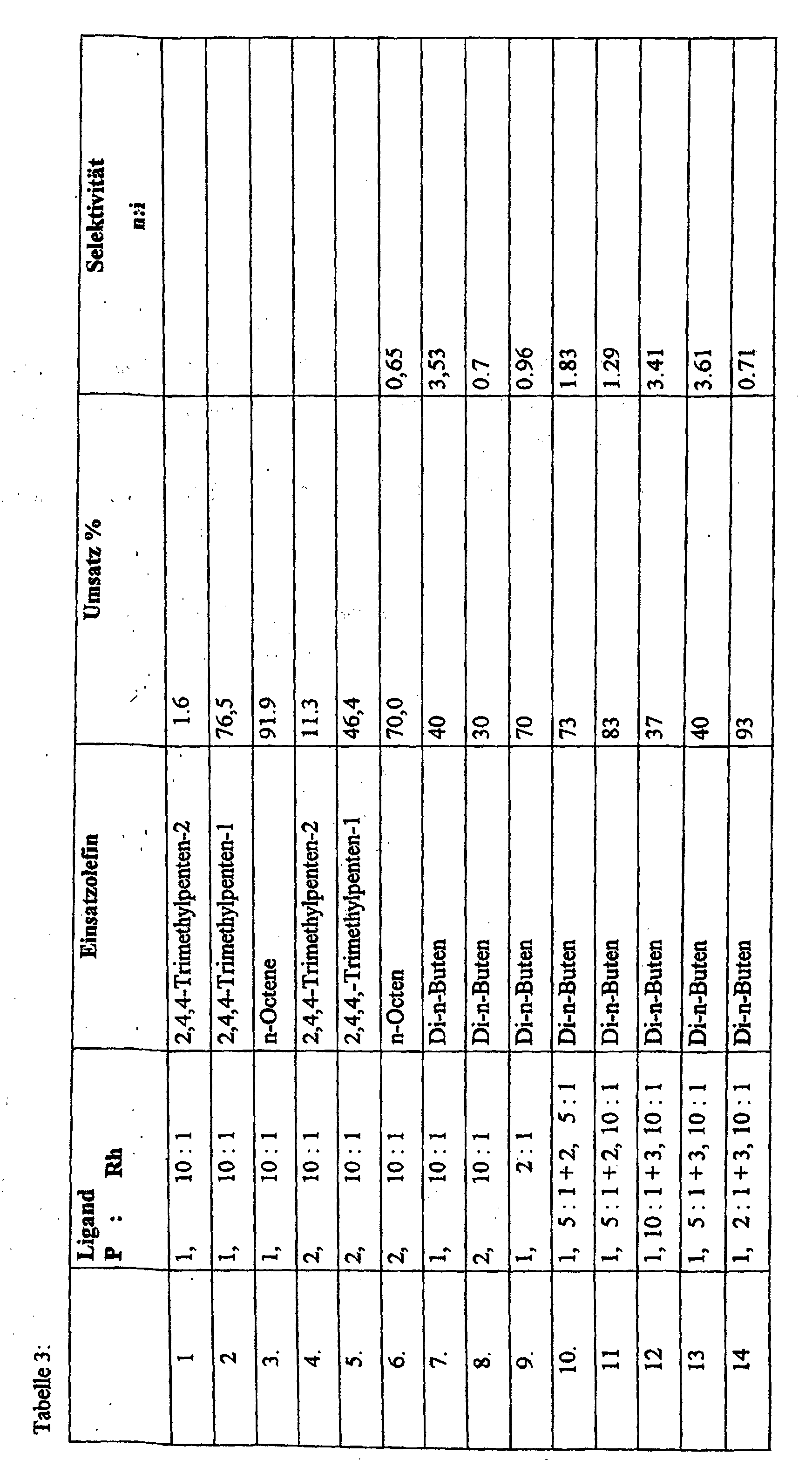

## Patentansprüche

1. Verfahren zur Herstellung von Aldehyden mit 4 bis 25 Kohlenstoffatomen durch katalytische Hydroformylierung der entsprechenden Olefine,
**dadurch gekennzeichnet,**
**daß** als Katalysator ein Metall der 8. Nebengruppe des Periodensystems in Gegenwart eines Liganden A der Formel I 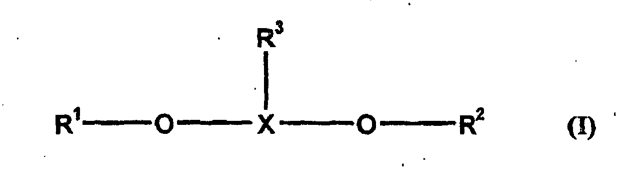 mit
X = As, Sb oder P und
R¹, R², R³: substituierter oder unsubstituierter aliphatischer, cycloaliphatischer oder aromatischer Kohlenwasserstoffrest mit 1 bis 50 Kohlenstoffatomen,
wobei zwei der Reste R¹, R², R³ eine kovalente Verknüpfung aufweisen können, mit der Maßgabe, daß wenigstens einer der Kohlenwasserstoffreste R¹, R², R³ ein Heteroatom aus der Gruppe O, S, N, F, Cl, Br, I, Se und Te enthält, und eines Liganden B der Formel II 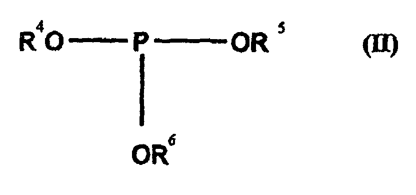 mit
R⁴, R⁵, R⁶: substituierter oder unsubstituierter aliphatischer oder aromatischer Kohlenwasserstoffrest mit 1 bis 50 Kohlenstoffatomen,
wobei je zwei der Reste R⁴, R⁵ und R⁶ eine kovalente Verknüpfung aufweisen können, verwendet wird.

2. Verfahren nach Anspruch 1,
**dadurch gekennzeichnet,**
**daß** ein Ligand A der Formel III 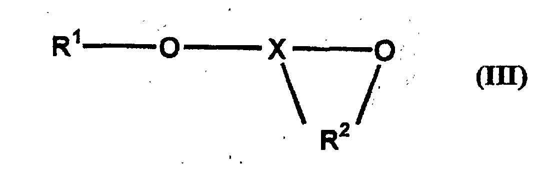 mit
X = As, Sb oder P und
R¹, R²: substituierter oder unsubstituierter aliphatischer, cycloaliphatischer oder aromatischer Kohlenwasserstoffrest mit 1 bis 50 Kohlenstoffatomen,
mit der Maßgabe, daß R¹ oder R² ein Heteroatom aus der Gruppe O, S, N, F, Cl, Br, I, Se und Te enthalten, verwendet wird.

3. Verfahren nach Anspruch 1,
**dadurch gekennzeichnet,**
**daß** ein Ligand A der Formel IV 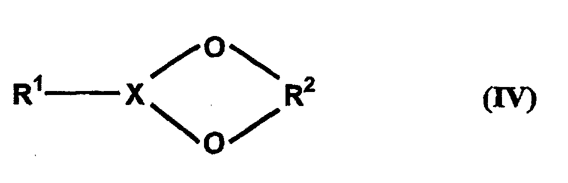
mit X = As, Sb oder P und
R¹, R²: substituierter oder unsubstituierter aliphatischer, cycloaliphatischer oder aromatischer Kohlenwasserstoffrest mit 1 bis 50 Kohlenstoffatomen,
mit der Maßgabe, daß R¹ oder R² ein Heteroatom der Gruppe O, S, N, F, Cl, Br, I, Se und Te enthalten, verwendet wird.

4. Verfahren nach Anspruch 1,
**dadurch gekennzeichnet,**
**daß** ein Ligand A der Formel V 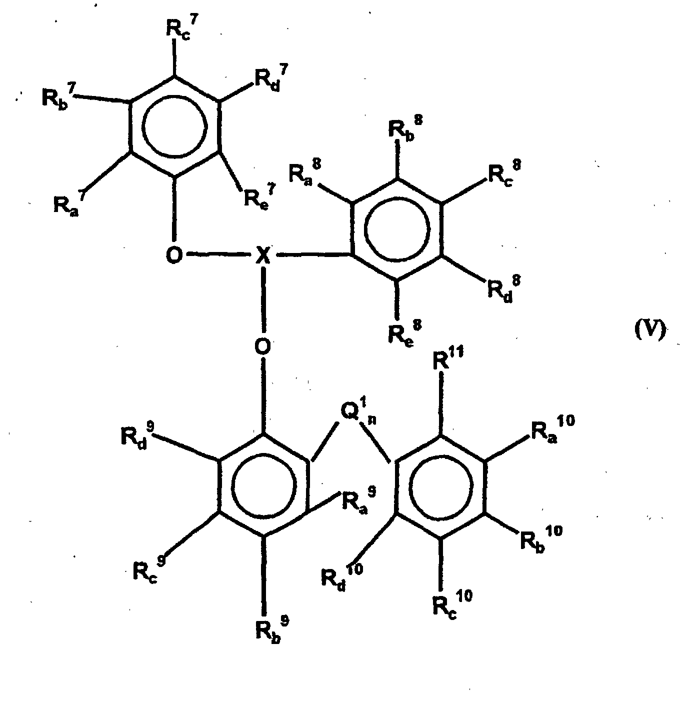 mit X = As, Sb oder P,
R⁷ ₐ₋ₑ, R⁸ ₐ₋ₑ, R⁹ _{a-d}, R¹⁰ _{a-d} = H, aliphatischer oder aromatischer Kohlenwasserstoffrest, aliphatische oder aromatische Alkoxygruppe, jeweils mit 1 bis 25 Kohlenstoffatomen, wobei die Substitutenten mit den Indices a-e jeweils für gleiche oder verschiedene Substituenten stehen können,
R¹¹ = -O-R¹², -CH₂-O-R¹², -COOR¹², -COOM, -SR¹², -NR¹²R^{13,} -CH₂NR¹²R¹³, -N=CR¹²R¹³, -CH₂COOM, wobei R¹² und R¹³ gleich oder verschieden sein können und die gleiche Bedeutung wie R⁷ ₐ besitzen und M = H,Li, Na, K, NH₄ bedeutet,
Q¹ = CR¹⁴R¹⁵, wobei R¹⁴ und R¹⁵ gleich oder verschieden sein können und die gleiche Bedeutung wie R⁷ ₐ besitzen und
n = 0 oder 1,
verwendet wird.

5. Verfahren nach Anspruch 1 oder 2,
**dadurch gekennzeichnet,**
**daß** ein Ligand A der Formel VI 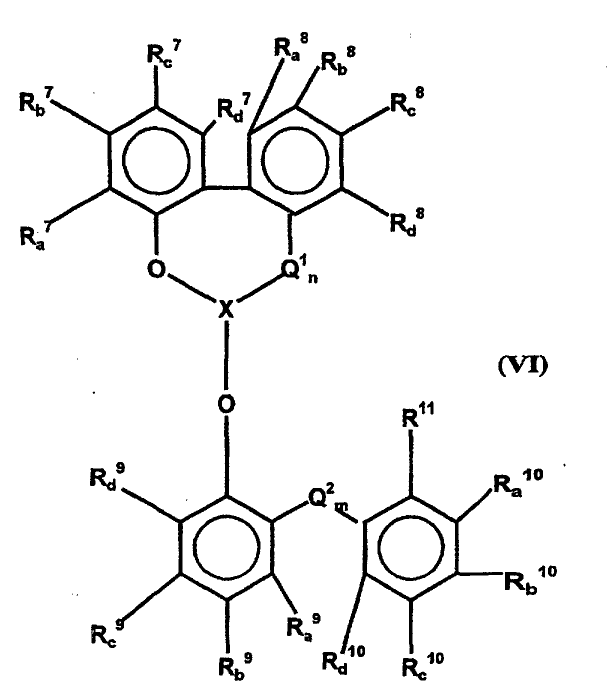 mit X = As, Sb oder P,
R⁷_{a-d}, R⁸_{a-d}, R⁹_{a-d}, R¹⁰_{a-d} = H, aliphatischer oder aromatischer Kohlenwasserstoffrest, aliphatische oder aromatische Alkoxygruppe jeweils mit 1 bis 25 Kohlenstoffatomen, wobei die Substituenten mit den Indices a-d jeweils für gleiche oder verschiedene Substituenten stehen können,
R¹¹ = -O-R¹², -CH₂-O-R¹², -COOR¹², -COOM, -SR¹², -NR¹²R^{13,} -CH₂NR¹²R¹³, -N=CR¹²R¹³, -CH₂COOM,
Q¹, Q² = CR¹⁴R¹⁵, wobei R¹², R¹³, R¹⁴ und R¹⁵ gleich oder verschieden sein können und die gleiche Bedeutung wie R⁷ₐ besitzen, M = H, Li, Na, K, NH₄ bedeutet und
n, m= 0 oder 1
verwendet wird.

6. Verfahren nach Anspruch 1 oder 3,
**dadurch gekennzeichnet,**
**daß** ein Ligand A der Formel VII 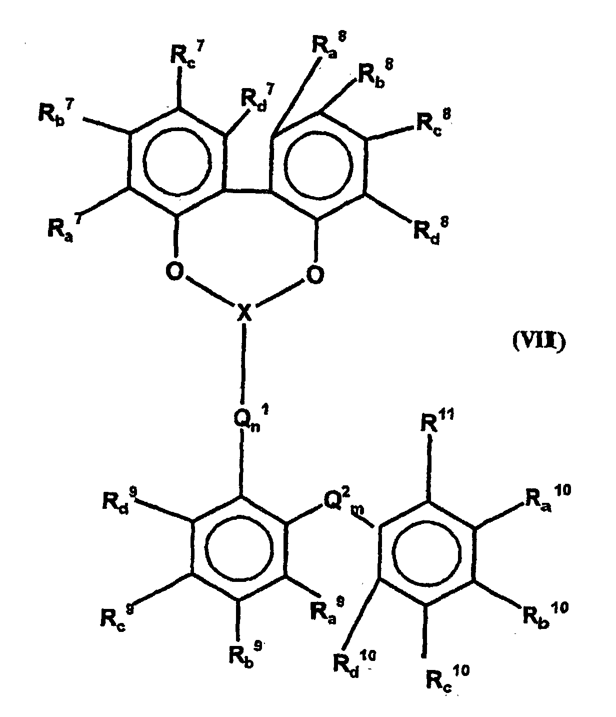 mit X = As, Sb oder P,
R⁷_{a-d}, R⁸_{a-d}, R⁹_{a-d}, R¹⁰_{a-d} = H, aliphatischer oder aromatischer Kohlenwasserstoffrest, aliphatische oder aromatische Alkoxygruppe jeweils mit 1 bis 25 Kohlenstoffatomen, wobei die Substituenten mit den Indices a-d jeweils für gleich oder verschiedene Substituenten stehen können,
R¹¹ = -O-R¹², -CH₂-O-R¹², -COOR¹², -COOM, -SR¹², -NR¹²R¹³, -CH₂NR¹²R¹³, -N=CR¹²R¹³, -CH₂CO₂M
Q¹, Q² = CR¹⁴R¹⁵, wobei R¹², R¹³, R¹⁴ und R¹⁵ gleich oder verschieden sein können und die gleiche Bedeutung wie R⁷ₐ besitzen, M = H, Na, K bedeutet und
n,m = 0 oder 1
verwendet wird.

7. Verfahren nach einem der Ansprüche 1 bis 6,
**dadurch gekennzeichnet,**
**daß** als Ligand B Verbindungen der Formel VIII 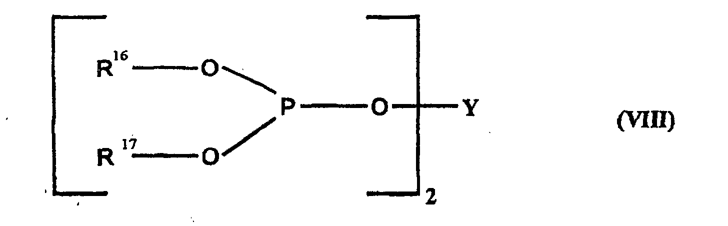 mit
R¹⁶, R¹⁷: aliphatischer oder aromatischer, substituierter oder unsubstituierter Kohlenwasserstoffrest mit 1 bis 50 Kohlenstoffatomen, wobei R¹⁶ und R¹⁷ gleich oder verschieden sein können und
Y: aliphatischer oder aromatischer, substituierter oder unsubstituierter Kohlenwasserstoffrest mit 1 bis 50 Kohlenstoffatomen
eingesetzt werden.

8. Verfahren nach einem der Ansprüche 1 bis 6,
**dadurch gekennzeichnet,**
**daß** als Ligand B Verbindungen der Formel IX 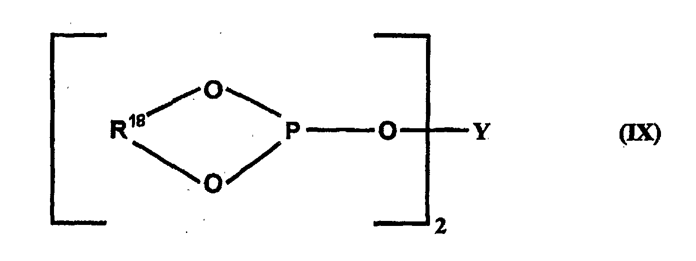 mit
R¹⁸: aliphatischer oder aromatischer, substituierter oder unsubstituierter Kohlenwasserstoffrest mit 1 bis 50 Kohlenstoffatomen und
Y: aliphatischer oder aromatischer, substituierter oder unsubstituierter Kohlenwasserstoffrest mit 1 bis 50 Kohlenstoffatomen
eingesetzt werden.

9. Verfahren nach einem der Ansprüche 1 bis 6,
**dadurch gekennzeichnet,**
**daß** als Ligand B Verbindungen der Formel X 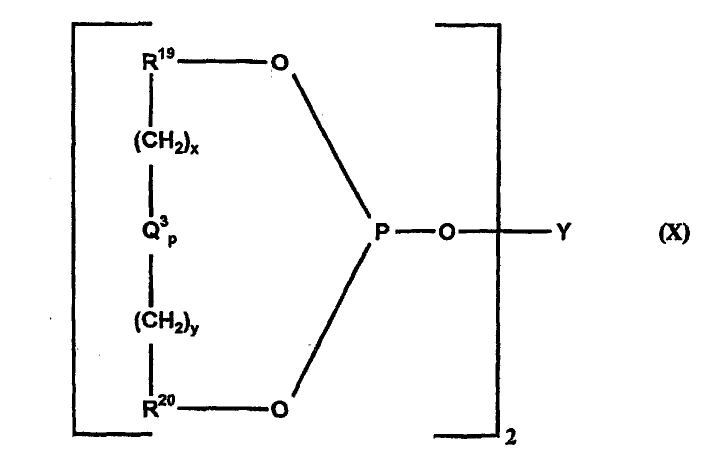 mit
R¹⁹, R²⁰: aliphatischer oder aromatischer, substituierter oder unsubstituierter Kohlenwasserstoffrest mit 1 bis 50 Kohlenstoffatomen, wobei R¹⁹ und R²⁰ gleich oder verschieden sein können,
Q³: C(R²¹)₂, O, S, NR²¹, Si(R²¹)₂, CO, wobei R²¹ = H, substituierter oder unsubstituierter aliphatischer oder aromatischer Kohlenwasserstoffrest mit 1 bis 50 Kohlenwasserstoffatomen,
Y: aliphatischer oder aromatischer, substituierter oder unsubstituierter Kohlenwasserstoffrest mit 1 bis 50 Kohlenstoffatomen und
x,y,p: 0 bis 5
eingesetzt werden.

10. Verfahren nach einem der Ansprüche 1 bis 6,
**dadurch gekennzeichnet,**
**daß** als Ligand B Verbindungen der Formel XI 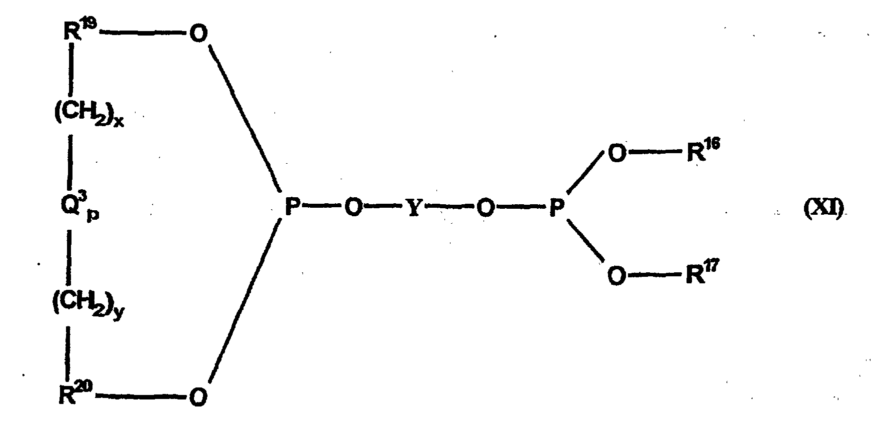 mit
R¹⁶, R¹⁷: aliphatischer oder aromatischer, substituierter oder unsubstituierter Kohlenwasserstoffrest mit 1 bis 50 Kohlenstoffatomen, wobei R¹⁶ und R¹⁷ gleich oder verschieden sein können,
R¹⁹, R²⁰: aliphatischer oder aromatischer, substituierter oder unsubstituierter Kohlenwasserstoffrest mit 1 bis 50 ' Kohlenstoffatomen, wobei R¹⁹ und R²⁰ gleich oder verschieden sein können,
Q³: C(R²¹)₂, O, S, NR²¹, Si(R²¹)₂, CO, wobei R²¹= H, substituierter oder unsubstituierter aliphatischer oder aromatischer Kohlenwasserstoffrest mit 1 bis 50 Kohlenwasserstoffatomen,
x,y,p: 0 bis 5 und
Y: aliphatischer oder aromatischer, substituierter oder unsubstituierter Kohlenwasserstoffrest mit 1 bis 50 Kohlenstoffatomen
eingesetzt werden.

11. Verfahren nach einem der Ansprüche 1 bis 6,
**dadurch gekennzeichnet,**
**daß** als Ligand B Verbindungen der Formel XII 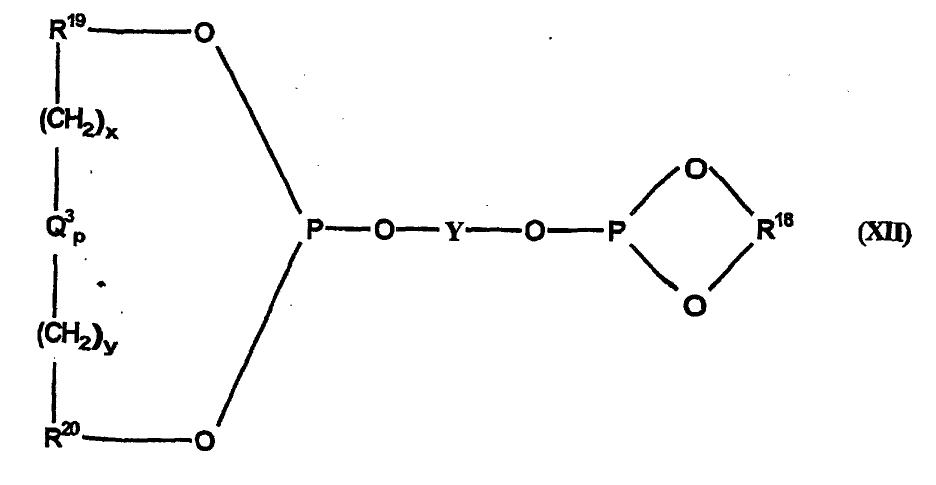 mit
R¹⁸, R¹⁹, R²⁰: aliphatischer oder aromatischer, substituierter oder unsubstituierter Kohlenwasserstoffrest mit 1 bis 50 Kohlenstoffatomen, wobei R¹⁸, R¹⁹, und R²⁰ gleich oder verschieden sein können,
Q³: C(R²¹)₂, O, S, NR²¹, Si(R²¹)₂, CO, wobei R²¹ = H, substituierter oder unsubstituierter aliphatischer oder aromatischer Kohlenwasserstoffrest mit 1 bis 50 Kohlenwasserstoffatomen,
x,y,p: 0 bis 5 und
Y: xaliphatischer oder aromatischer, substituierter oder unsubstituierter Kohlenwasserstoffrest mit 1 bis 50 Kohlenstoffatomen
eingesetzt werden.

12. Verfahren nach einem der Ansprüche 1 bis 6,
**dadurch gekennzeichnet,**
**daß** als Ligand B Verbindungen der Formel XIII 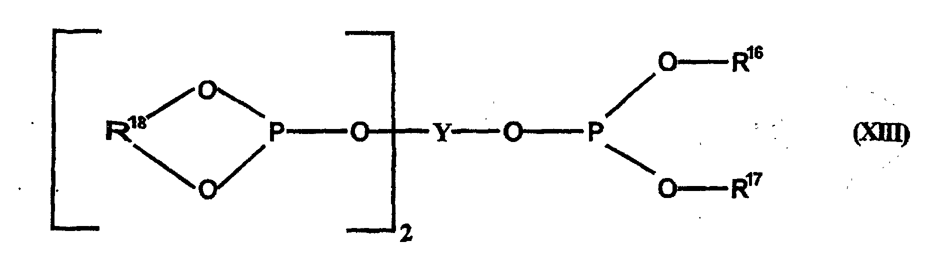 mit
R¹⁶, R¹⁷, R¹⁸: aliphatischer oder aromatischer, substituierter oder unsubstituierter Kohlenwasserstoffrest mit 1 bis 50 Kohlenstoffatomen, wobei R¹⁶, R¹⁷ und R¹⁸ gleich oder verschieden sein können und
Y: aliphatischer oder aromatischer, substituierter oder unsubstituierter Kohlenwasserstoffrest mit 1 bis 50 Kohlenstoffatomen
eingesetzt werden.

## Claims

1. A process for preparing aldehydes having from 4 to 25 carbon atoms by catalytic hydroformylation of the corresponding olefins, **characterized in that** the catalyst used comprises a metal of transition group 8 of the Periodic Table in the presence of a ligand A of the formula I 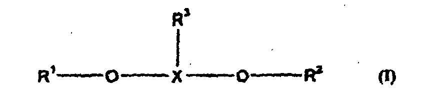 where X = As, Sb or P and
R¹, R², R³: are each a substituted or unsubstituted aliphatic, cycloaliphatic or aromatic hydrocarbon radical having from 1 to 50 carbon atoms,
where two of the radicals R¹, R², R³ can be covalently linked with the proviso that at least one of the hydrocarbon radicals R¹, R², R³ contains a heteroatom selected from the group consisting of O, S, N, F, Cl, Be, I, Se and Te, and a ligand B of the formula II 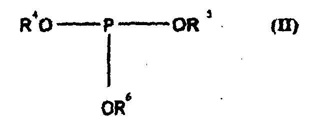 where
R⁴,R⁵,R⁶: are each a substituted or unsubstituted aliphatic or aromatic hydrocarbon having from 1 to 50 carbon atoms,
where two of the radicals R⁴, R⁵ and R⁶ can be covalently linked.

2. A process according to claim 1, **characterized in that** the ligand A used has the formula III 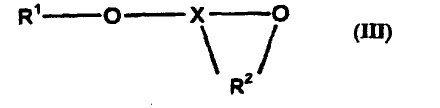 where X = As, Sb or P and
R¹, R² are substituted or unsubstituted, aliphatic, cycloaliphatic or aromatic hydrocarbon radicals having from 1 to 50 carbon atoms,
with the proviso that R¹ or R² contains a heteroatom selected from the group consisting of O, S, N, F, Cl, Be, I, Se and Te.

3. A process according to claim 1, **characterized in that** the ligand A used has the formula IV 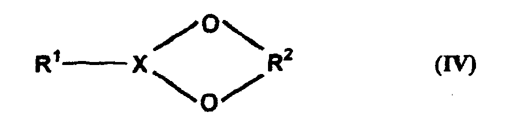 where X = As, Sb or P and
R¹, R² are substituted or unsubstituted, aliphatic, cycloaliphatic or aromatic hydrocarbon radicals having from 1 to 50 carbon atoms,
with the proviso that R¹ or R² contains a heteroatom selected from the group consisting of O, S, N, F, Cl, Be, I, Se and Te.

4. A process according to claim 1, **characterized in that** the ligand A used has the formula V 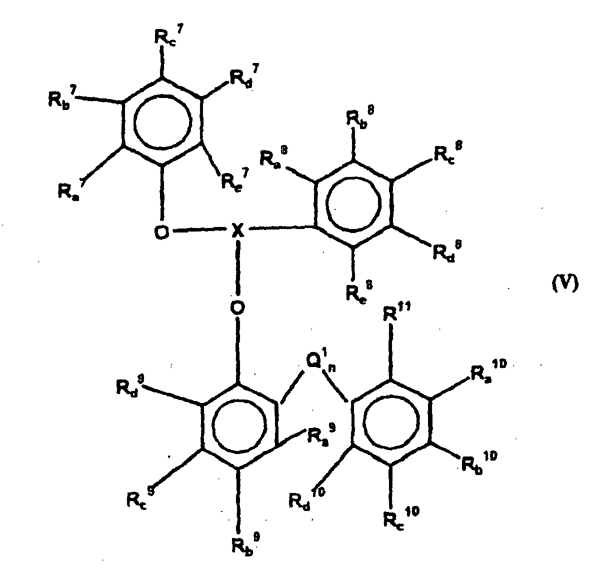 where X = As, Sb or P,
R⁷ₐ₋ₑ, R⁸ₐ₋ₑ, R⁹_{a-d} and R¹⁰_{a-d} = H, aliphatic or aromatic hydrocarbon radicals, aliphatic or aromatic alkoxy groups, each having from 1 to 25 carbon atoms, where the substituents with the indices a-e may in each case be identical or different.
R¹¹ = -O-R¹², -CH₂-O-R¹², -COOR¹², -COOM, -SR¹², -NR¹²R¹³, -CH₂NR¹²R¹³, -N=CR¹²R¹³, -CH₂COOM, where R¹² and R¹³ may be identical or different and are as defined for R⁷ₐ and M = H, Li, Na, K, NH₄,
Q¹ = CR¹⁴R¹⁵, where R¹⁴ and R¹⁵ may be identical or different and are as defined for R⁷ₐ and
n = 0 or 1.

5. A process according to claim 1 or 2, **characterized in that** the ligand A used has the formula VI 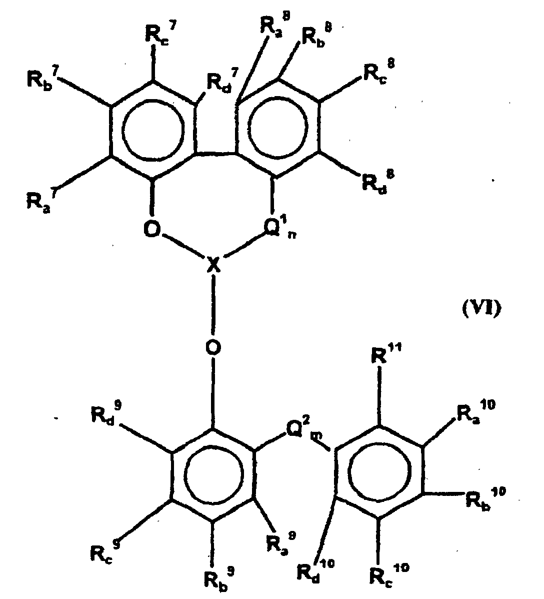 where X = As, Sb or P,
R⁷_{a-d}, R⁸_{a-d}, R⁹_{a-d} and R¹⁰_{a-d} = H, aliphatic or aromatic hydrocarbon radicals, aliphatic or aromatic alkoxy groups, each having from 1 to 25 carbon atoms, where the substituents with the indices a-d may in each case be identical or different,
R¹¹ = -O-R¹², -CH₂-O-R¹², -COOR¹², -COOM, -SR¹², -NR¹²R¹³, -CH₂NR¹²R¹³, -N=CR¹²R¹³, -CH₂COOM,
Q¹,Q² = CR¹⁴R¹⁵, where R¹², R¹³, R¹⁴ and R¹⁵ may be identical or different and are as defined for R⁷ₐ and M = H, Li, Na, K, NH₄ and
n,m = 0 or 1.

6. A process according to claim 1 or 3, **characterized in that** the ligand A used has the formula VII 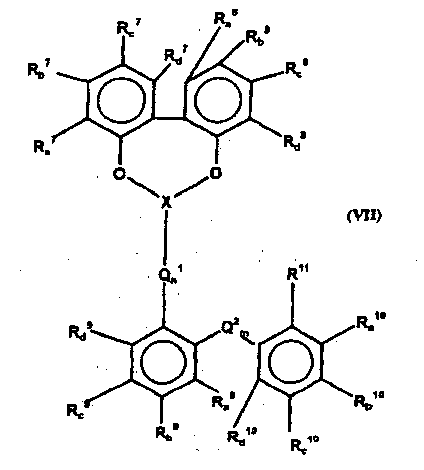 where X = As, Sb or P,
R⁷_{a-d}, R⁸_{a-d}, R⁹_{a-d} and R¹⁰_{a-d} = H, aliphatic or aromatic hydrocarbon radicals, aliphatic or aromatic alkoxy groups, each having from 1 to 25 carbon atoms, where the substituents with the indices a-d may in each case be identical or different,
R¹¹ = -O-R¹², -CH₂-O-R¹², -COOR¹², -COON, -SR¹², -NR¹²R¹³, -CH₂NR¹²R¹³, -N=CR¹²R¹³, -CH₂CO₂M,
Q¹,Q² = xCR¹⁴R¹⁵, where R¹², R¹³, R¹⁴ and R¹⁵ may be identical or different and are as defined for R⁷ₐ and M = H, Na, K and
n,m = 0 or 1.

7. A process according to any of claims 1 to 6, **characterized in that** the ligand B used is a compound of the formula VIII 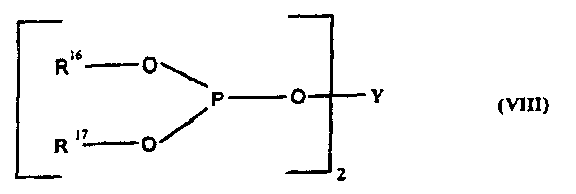 where
R¹⁶,R¹⁷: aliphatic or aromatic, substituted or unsubstituted hydrocarbon radicals having from 1 to 50 carbon atoms, where R¹⁶ and R¹⁷ may be identical or different, and
Y: an aliphatic or aromatic, substituted or unsubstituted hydrocarbon radical having from 1 to 50 carbon atoms.

8. A process according to any of claims 1 to 6, **characterized in that** the ligand B used is a compound of the formula IX 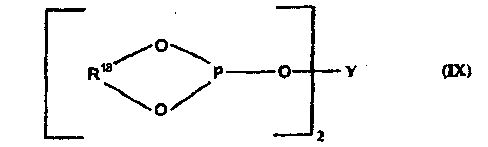 where
R¹⁸: an aliphatic or aromatic, substituted or unsubstituted hydrocarbon radical having from 1 to 50 carbon atoms and
Y: an aliphatic or aromatic, substituted or unsubstituted hydrocarbon radical having from 1 to 50 carbon atoms.

9. A process according to any of claims 1 to 6, **characterized in that** the ligand B used is a compound of the formula X 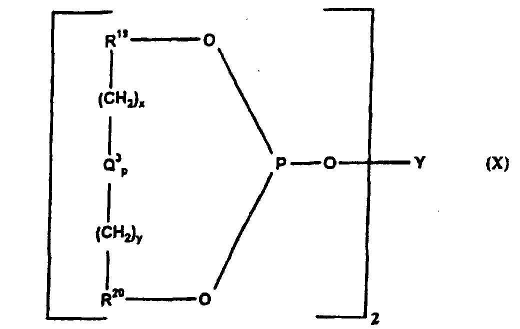 where
R¹⁹, R²⁰: aliphatic or aromatic, substituted or unsubstituted hydrocarbon radicals having from 1 to 50 carbon atoms, where R¹⁹ and R²⁰ may be identical or different,
Q³: C(R²¹)₂, O, S, NR²¹, Si(R²¹)₂, CO, where R²¹ = H or a substituted or unsubstituted aliphatic or aromatic hydrocarbon radical having from 1 to 50 carbon atoms,
Y: an aliphatic or aromatic, substituted or unsubstituted hydrocarbon radical having from 1 to 50 carbon atoms and
x,y,p: from 0 to 5.

10. A process according to any of claims 1 to 6, **characterized in that** the ligand B used is a compound of the formula XI 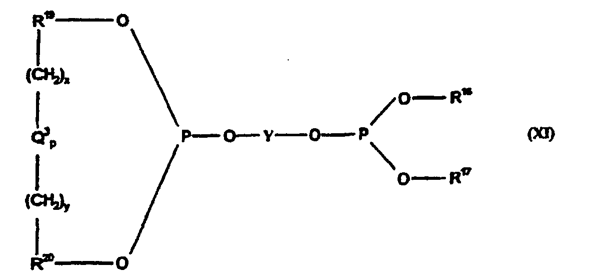 where
R¹⁶, R¹⁷: aliphatic or aromatic, substituted or unsubstituted hydrocarbon radicals having from 1 to 50 carbon atoms, where R¹⁶ and R¹⁷ may be identical or different,
R¹⁹,R²⁰: aliphatic or aromatic, substituted or unsubstituted hydrocarbon radicals having from 1 to 50 carbon atoms, where R¹⁹ and R²⁰ may be identical or different,
Q³: C(R²¹)₂, O, S, NR²¹, Si(R²¹)₂, CO, where R²¹ = H or a substituted or unsubstituted aliphatic or aromatic hydrocarbon radical having from 1 to 50 carbon atoms,
x,y,p: from 0 to 5 and
Y: an aliphatic or aromatic, substituted or unsubstituted hydrocarbon radical having from 1 to 50 carbon atoms.

11. A process according to any of claims 1 to 6, **characterized in that** the ligand B used is a compound of the formula XII 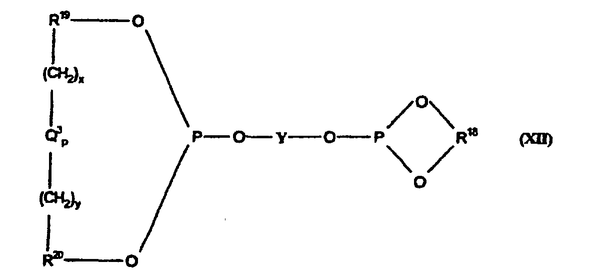 where
R¹⁸,R¹⁹,R²⁰: aliphatic or aromatic, substituted or unsubstituted hydrocarbon radicals having from 1 to 50 carbon atoms, where R¹⁸, R¹⁹ and R²⁰ may be identical or different,
Q³: C(R²¹)₂, O, S, NR²¹, Si(R²¹)₂, CO, where R²¹ = H or a substituted or unsubstituted aliphatic or aromatic hydrocarbon radical having from 1 to 50 carbon atoms,
x,y,p: from 0 to 5 and
Y: an aliphatic or aromatic, substituted or unsubstituted hydrocarbon radical having from 1 to 50 carbon atoms.

12. A process according to any of claims 1 to 6, **characterized in that** the ligand B used is a compound of the formula XIII 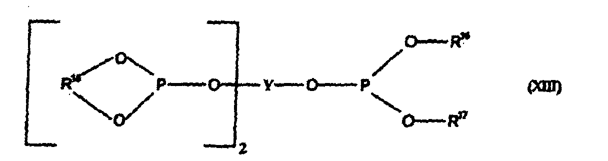 where
R¹⁶, R¹⁷, R¹⁸ aliphatic or aromatic, substituted or unsubstituted hydrocarbon radicals having from 1 to 50 carbon atoms, where R¹⁶, R¹⁷ and R¹⁸ may be identical or different, and
Y: an aliphatic or aromatic, substituted or unsubstituted hydrocarbon radical having from 1 to 50 carbon atoms.

## Revendications

1. Procédé de production d'aldéhydes ayant de 4 à 25 atomes de carbone par hydroformylation catalytique des oléfines correspondants,
**caractérisé en ce que**
comme catalyseur on utilise un métal du 8^{ème} sous groupe du système périodique en présence d'un Ligand A de Formule 1 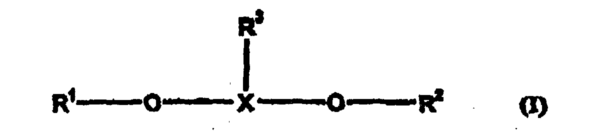 avec
X = As, Sb ou P et
R¹, R², R³ : représentent un reste d'hydrocarbure substitué ou non substitué, aliphatique, cycloaliphatique ou aromatique ayant de 1 à 50 atomes de carbone,
dans lequel deux des restes R¹, R², R³ peuvent posséder une liaison covalente, avec la restriction qu'au moins un des restes d'hydrocarbures R¹, R², R³ renferme un hétéroatome choisi dans le groupe de O, S, N, F, Cl, Br, I, Se et Te et en présence d'un ligand B de formule II 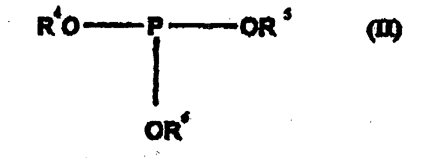 R⁴, R⁵, R⁶ étant des restes carbonés substitués ou non substitués, aliphatiques ou aromatiques ayant de 1 à 5 atomes de carbone,
dans laquelle deux des restes R⁴, R⁵, R⁶ peuvent posséder une liaison covalente.

2. Procédé selon la revendication 1,
**caractérisé en ce qu'**
un ligand A de formule III 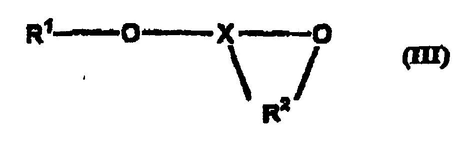 avec
X = As, Sb ou P et
R¹, R² : étant des restes d'hydrocarbures substitués ou non substitués, allphatique, cycloaliphatique, ou aromatique ayant de 1-50 atomes de carbone, avec la restriction que
R¹ ou R² renferment un hétéroatome du groupe de O, S, N, F ; Cl, Br, I, Se et Te.

3. Procédé selon la revendication 1,
**caractérisé en ce qu'**
on utilise un ligand A de formule IV 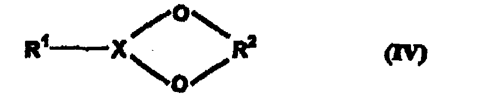 avec
X = As, Sb ou P et
R¹, R² : sont des restes d'hydrocarbures substitués ou non substitués, aliphatiques, cycloaliphatiques ou aromatiques ayant de 1 à 50 atomes de carbone avec la restriction que
R¹ ou R² renferment un hétéroatome du groupe de O, S, N, F, Cl, F, I, Se et Te.

4. Procédé selon la revendication 1
**caractérisé en ce qu'**
on utilise un ligand A de formule V 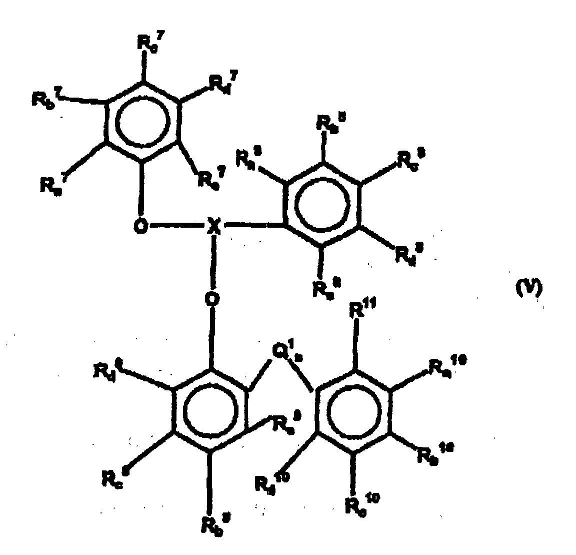
avec X = As, Sb ou P
R⁷a-e, R⁸a-e, R⁹a-d, R¹⁰a-d = H sont égaux à un reste hydrocarboné aliphatique ou aromatique, un groupe alkoxyaliphatique ou aromatique, respectivement avec 1 à 25 atomes de carbone, dans lesquels les substituants ayant les indices a-e peuvent représenter à chaque fois des substituants identiques ou différents,
R¹¹ est égal à
-O-R¹², -CH₂-O-R¹², -COOR¹², -COOM, -SR¹², -NR¹²R¹³, -CH₂NR¹²R¹³, -CH₂NR¹²R¹³, N=CR¹²R¹³,
-CH₂COOM, dans laquelle R¹² et R¹³ peuvent être identiques ou différents et possèdent la même signification que R⁷a, et M signifie H, Li, Na, K ou NH₄,
Q¹ est un groupe CR¹⁴R¹⁵ dans lequel R¹⁴ et R¹⁵ peuvent être identiques ou différents et possèdent la même signification que R⁷a et
n = 0 ou 1.

5. Procédé selon la revendication 1 ou la revendication 2,
**caractérisé en ce qu'**
on utilise un ligand A de formule VI 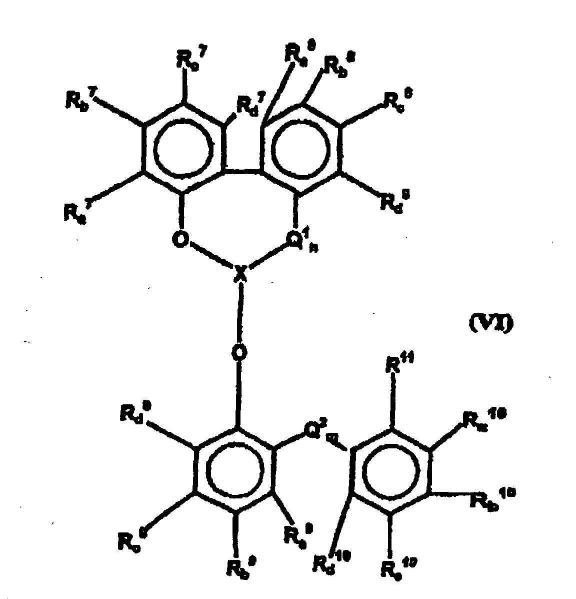
X = As, Sb ou P,
R⁷a-d, R⁸a-d, R⁹a-d, R¹⁰a-d et H, un reste hydrocarboné aliphatique ou aromatique avec respectivement de 1 à 25 atomes de carbone dans lequel les substituants ayant les indices a-d respectivement peuvent représenter des substituants identiques ou différents
R¹¹ est égal à
-O-R¹², -CH₂-O-R¹², -COOR¹², -COOM, -SR¹², -NR¹²R¹³, -CH₂NR¹²R¹³, -CH₂NR¹²R¹³, N=CR¹²R¹³, -CH₂COOM,
Q¹, Q² est égal à
CR¹⁴R¹⁵, dans lesquels R¹², R¹³, R¹⁴ et R¹⁵ peuvent être identiques ou différents et possèdent la même signification que R⁷a, M signifie H, Li, Na, K ou NH₄ et n, m sont égaux à 0 ou 1.

6. Procédé selon la revendication 1 ou la revendication 3,
**caractérisé en ce qu'**
on utilise un ligand A de formule VII 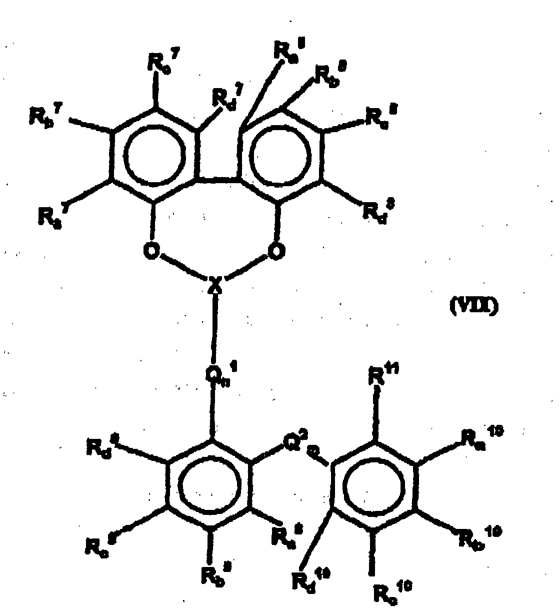
avec X = As, Sb ou P,
R⁷a-d, R⁸a-d, R⁹a-d, R¹⁰a-d soit H, un reste hydrocarboné aliphatique ou aromatique, un groupe alkoxyaliphatique ou aromatique respectivement avec de 1 à 25 atomes indices a-d peuvent représenter respectivement des substituants identiques ou différents
R¹¹ signifie
-O-R¹², -CH₂-O-R¹², -COOR¹², -COOM, -SR¹², -NR¹²R¹³, -CH₂NR¹²R¹³,-N=CR¹²R¹³, ou -CH₂CO₂M,
Q¹, Q² signifient CR¹⁴R¹⁵, où R¹², R¹³, R¹⁴ et R¹⁵ peuvent être identiques ou différents et possèdent la même signiflcation que R⁷a et M signifie H, Na, K,
n, m sont égaux à 0 ou 1.

7. Procédé selon l'une des revendications 1 à 6,
**caractérisé en ce qu'**
on met en oeuvre comme ligand A des composés de formule VIII 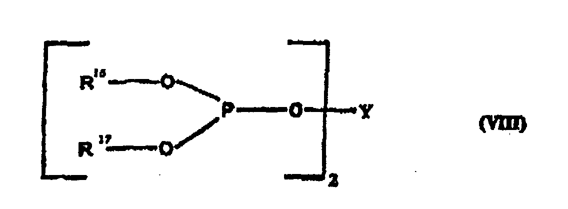 avec
R¹⁶, R¹⁷ représentent un reste d'hydrocarbure aliphatique ou aromatique, substitué ou non substitué ayant de 1 à 50 atomes de carbone dans lesquels R¹⁶ et R¹⁷ peuvent être identiques ou différents,
et Y signifie un reste d'hydrocarbure aliphatique ou aromatique, substitué ou non substitué, ayant de 1 à 50 atomes de carbone.

8. Procédé selon l'une des revendications 1 à 6,
**caractérisé en ce qu'**
on met en oeuvre comme ligand B, des composés de formule IX 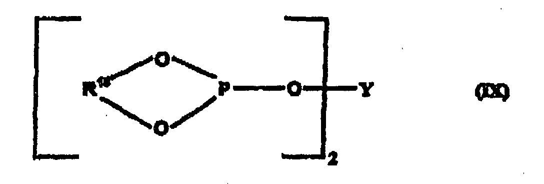 avec
R¹⁸ représente un reste d'hydrocarbure aliphatique ou aromatique, substitué ou non substitué ayant de 1 à 50 atomes de carbone,
Y représente un reste d'hydrocarbure aliphatique ou aromatique, substitué ou non substitué ayant de 1 à 50 atomes de carbone.

9. Procédé selon l'une des revendications 1 à 6,
**caractérisé en ce qu'**
on met en oeuvre comme ligand B des composés de formule X 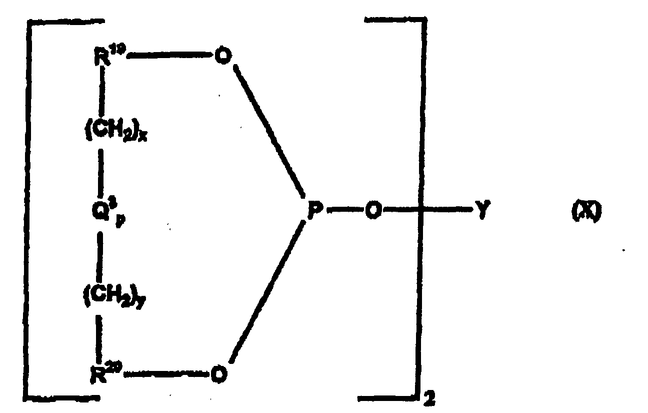 avec
R¹⁹, R²⁰ représentant un reste d'hydrocarbure aliphatique ou aromatique, substitué ou non substitué ayant de 1 à 50 atomes de carbone, dans lesquels R¹⁹ et R²⁰ peuvent être identiques ou différents
Q³ représente C(R²¹)₂, O, S, NR²¹, Si(R²¹)₂, CO, dans lesquels R²¹ = H, un reste d'hydrocarbure substitué ou non substitué, aliphatique ou aromatique ayant de 1 à 5 atomes de carbone
Y représente un reste d'hydrocarbure aliphatique ou aromatique, substitué ou non substitué ayant de 1 à 50 atomes de carbone
x, y, p sont égaux à 0 à 5.

10. Procédé selon l'une des revendications 1 à 6,
**caractérisé en ce qu'**
on met en oeuvre comme ligand B, des composés de formule XI 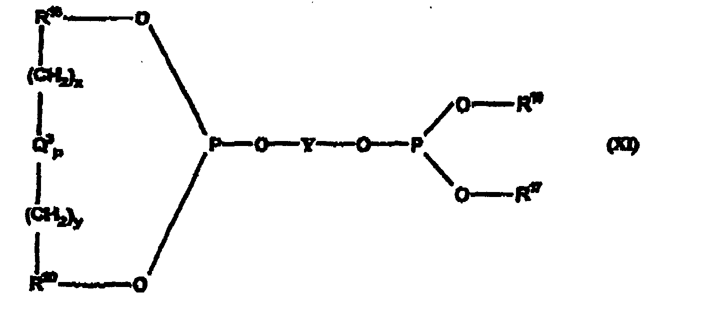 avec
R¹⁶, R¹⁷ représentant un reste d'hydrocarbure aliphatique ou aromatique, substitué ou non substitué ayant de 1 à 50 atomes de carbone dans lesquels R¹⁶ et R¹⁷ peuvent être identiques ou différents,
R¹⁹, R²⁰ représentent un reste d'hydrocarbure aliphatique ou aromatique, substitué ou non substitué ayant de 1 à 50 atomes de carbone dans lesquels R¹⁹ et R²⁰ peuvent être identiques ou différents
Q³ représente C(R²¹)₂, O, S, NR²¹, Si(R²¹)₂, CO, dans lesquels R²¹ = H, un reste d'hydrocarbure substitué ou non substitué, aliphatique ou aromatique ayant de 1 à 50 atomes de carbone,
x, y et p représentent de 0 à 5
Y représente un reste d'hydrocarbure aliphatique ou aromatique substitué ou non substitué ayant de 1 à 50 atomes de carbone.

11. Procédé selon l'une des revendications 1 à 6,
**caractérisé en ce qu'**
on met en oeuvre comme ligand B des composés de formule XII 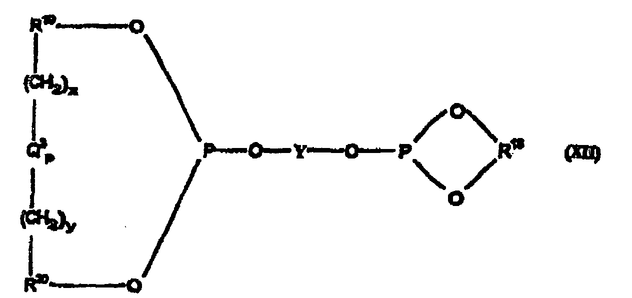
R¹⁸, R¹⁹ et R²⁰ représentant un reste d'hydrocarbure aliphatique ou aromatique, substitué ou non substitué ayant de 1 à 50 atomes de carbone, dans lesquels R¹⁸, R¹⁹ et R²⁰ peuvent être identiques ou différents
Q³ représente C(R²¹)₂, O, S, NR²¹, Si(R²¹)₂, CO, dans lesquels R²¹ = H, un reste d'hydrocarbure substitué ou non substitué, aliphatique ou aromatique ayant de 1 à 50 atomes de carbone,
x, y et p représentent de 0 à 5 et
Y représente un reste d'hydrocarbure aliphatique ou aromatique substitué ou non substitué ayant de 1 à 50 atomes de carbone.

12. Procédé selon l'une des revendications 1 à 6,
**caractérisé en ce qu'**
on met en oeuvre comme ligand B des composés de formule XIII 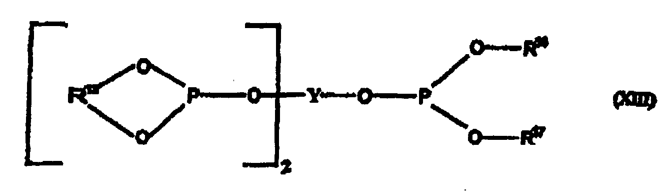
avec R¹⁶, R¹⁷ et R¹⁸ représentant un reste d'hydrocarbure aliphatique ou aromatique, substitué ou non substitué, ayant de 1 à 50 atomes de carbone, R¹⁶, R¹⁷, et R¹⁸ pouvant être identiques ou différents
et
Y représente un reste d'hydrocarbure aliphatique ou aromatique, substitué ou non substitué ayant de 1 à 50 atomes de carbone.
